(19) Europäisches Patentamt
European Patent Office
Office européen des brevets

(11) **EP 3 655 520 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**31.12.2025 Patentblatt 2026/01**

(45) Hinweis auf die Patenterteilung:
**06.09.2023 Patentblatt 2023/36**

(21) Anmeldenummer: **18746662.8**

(22) Anmeldetag: **23.07.2018**

(51) Internationale Patentklassifikation (IPC):
**C12N 1/14** (2006.01)    **C12N 1/20** (2006.01)
**C12P 39/00** (2006.01)    **C12P 19/42** (2006.01)

(52) Gemeinsame Patentklassifikation (CPC):
**C12P 19/42; C12P 39/00**

(86) Internationale Anmeldenummer:
**PCT/EP2018/069920**

(87) Internationale Veröffentlichungsnummer:
**WO 2019/016407 (24.01.2019 Gazette 2019/04)**

(54) **SEQUENTIELLES CO-KULTIVIERUNGSVERFAHREN ZUR HERSTELLUNG EINES VITAMIN- UND PROTEINREICHEN NAHRUNGSMITTELS**

SEQUENTIAL CO-CULTURING METHOD FOR PRODUCING A VITAMIN- AND PROTEIN-RICH FOOD PRODUCT

PROCÉDÉ DE CO-CULTURE SÉQUENTIEL POUR PRODUIRE UN ALIMENT RICHE EN VITAMINES ET EN PROTÉINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.07.2017 DE 102017212564**

(43) Veröffentlichungstag der Anmeldung:
**27.05.2020 Patentblatt 2020/22**

(73) Patentinhaber: **Frettlöh, Martin**
**57080 Siegen (DE)**

(72) Erfinder:
• **FRETTLÖH, Martin**
**57080 Siegen (DE)**
• **HAAG, Tanja**
**57076 Siegen (DE)**
• **ZORN, Holger**
**35435 Wettenberg (DE)**
• **ZAJUL, Martina**
**57076 Siegen (DE)**
• **AHLBORN, Jenny**
**35394 Gießen (DE)**

(74) Vertreter: **Berger, Axel Bernhard**
**Bardehle Pagenberg Partnerschaft mbB**
**Patentanwälte, Rechtsanwälte**
**Prinzregentenplatz 7**
**81675 München (DE)**

(56) Entgegenhaltungen:
**WO-A1-2007/139321      WO-A1-2015/169967**
**WO-A1-2016/113744**

• **ANONYMOUS: "Zusammensetzung Stutenmilch pro 100 ml - Stutenmolkerei de Lage Wierde", 1 January 2002 (2002-01-01), XP55861523, Retrieved from the Internet <URL:https://www.kraftderstutenmilch.de/informationen/zusammensetzung-stutenmilch/zusammensetzung-stutenmilch-pro-100-ml/> [retrieved on 20211115]**
• **CSAPO-KISS Z ET AL: "COMPOSITION OF MARES' COLOSTRUM AND MILK. PROTEIN CONTENT, AMINO ACID COMPOSITION AND CONTENTS OF MACRO- AND MICRO-ELEMENTS", INTERNATIONAL DAIRY JOURNAL, ELSEVIER APPLIED SCIENCE, BARKING, GB, vol. 5, no. 4, 1 January 1995 (1995-01-01), pages 403 - 415, XP001151880, ISSN: 0958-6946, DOI: 10.1016/0958-6946(94)00014-G**
• **THI LE NGUYEN ET AL: "Growth of Lactobacillus paracasei ssp. paracasei on tofu whey", INTERNATIONAL JOURNAL OF FOOD MICROBIOLOGY, ELSEVIER BV, NL, vol. 89, no. 1, 15 December 2003 (2003-12-15), pages 67 - 75, XP002498965, ISSN: 0168-1605, DOI: 10.1016/S0168-1605(3)00109-0**

- SELINA HUGENSCHMIDT ET AL: "Concurrent high production of natural folate and vitamin B12 using a co-culture process withSM39 andDF13", PROCESS BIOCHEMISTRY, ELSEVIER LTD, GB, vol. 46, no. 5, 18 January 2011 (2011-01-18), pages 1063 - 1070, XP028372433, ISSN: 1359-5113, [retrieved on 20110125], DOI: 10.1016/ J.PROCBIO.2011.01.021
- STEPHAN ALEXANDER ET AL: "Edible mushroom mycelia ofPleurotus sapidusas novel protein sources in a vegan boiled sausage analog system: functionality and sensory tests in comparison to commercial proteins and meat sausages", EUROPEAN FOOD RESEARCH AND TECHNOLOGY, SPRINGER BERLIN HEIDELBERG, BERLIN/HEIDELBERG, vol. 244, no. 5, 23 November 2017 (2017-11-23), pages 913 - 924, XP036482304, ISSN: 1438-2377, [retrieved on 20171123], DOI: 10.1007/S00217-017-3012-1

**Beschreibung**

Die vorliegende Erfindung betrifft ein Verfahren zur Herstellung eines vitamin- und proteinreichen Produkts, ein vitamin- und proteinreiches Produkt, welches gemäß dem Verfahren herstellbar ist, sowie Nahrungsmittel, welche dieses Produkt enthalten.

**1. Stand der Technik**

Angesichts der stetig wachsenden Weltbevölkerung und der gleichzeitig knapper werdenden Ressourcen, zum Beispiel von landwirtschaftlich nutzbaren Flächen, besteht ein Bedarf an hochwertigen, protein- und vitaminreichen Nahrungsmitteln aus neuartigen Nahrungsquellen. Bereits heute beträgt laut Welthunger-Index der Anteil an unterernährten Menschen in den Entwicklungsländern zwischen 1% und 70%. Nach Schätzungen der Welternährungsorganisation (FAO, Food and Agriculture Organisation) müssten die Erträge der Agrarwirtschaft entsprechend des weltweit zunehmenden Bedarfs an Lebensmitteln bis 2050 um 70% gesteigert werden, um eine ausreichende Versorgung sicherzustellen.

Zur ausreichenden Deckung des Proteinbedarfs werden heutzutage vor allem tierische Lebensmittel eingesetzt. Deren Produktion weist jedoch eine ungünstige Energiebilanz auf: Um beispielsweise 1 kg Schweinefleisch zu produzieren, werden 4900 L Wasser, 4 kg Futter, 9,9 $m^2$ Platzbedarf, eine Mast von mindestens einem halben Jahr mit anschließender 3-4 Tage umfassender Schlachtung bei gleichzeitigem $CO_2$-Ausstoß von 8,5 kg benötigt. Ein weiteres Problem der Produktion tierischer Produkte liegt im verbreiteten Antibiotikaeinsatz in der Tierhaltung, der zu Resistenzbildung von Bakterien und damit zur Unwirksamkeit von Antibiotika führen kann.

Vegetarische proteinreiche Produkte, die in ihrer äußeren Form Fleischprodukten ähneln, basieren häufig auf Sojaprotein. Ein wesentlicher Nachteil in der Verwendung von Soja liegt in der Notwendigkeit großer landwirtschaftlicher Nutzflächen zum Anbau. Auch werden zunehmend gentechnisch veränderte Sojapflanzen angebaut. Des Weiteren besitzen Produkte, die auf Soja, insbesondere auf gepresstem Soja basieren, keinen fleischähnlichen Geschmack oder Textur. Das Imitieren der Faserstruktur erfolgt daher mit Hilfe von Weizengluten, welches jedoch als Allergen zu Unverträglichkeiten führen kann. Sojahaltige Produkte können auch nicht den Vitamin-Bedarf vegetarisch oder vegan lebender Konsumenten decken, da insbesondere weder Vitamin D noch Vitamin B12 in der Sojabohne enthalten ist.

Weiterhin wurden Fleischimitate auf Milchproteinbasis entwickelt, die jedoch einerseits einen hohen Bedarf an landwirtschaftlichen Tiernutzflächen haben, andererseits aufgrund des hohen Milcheiweißgehalts und Laktosegehalts zu Unverträglichkeiten führen können.

Auch wurden Produkte auf Pilzbasis, insbesondere Schimmelpilze (Ascomyceten, z.B. *Fusarium venenatum*) entwickelt. EP1094719B1 beschreibt ein Verfahren zur Herstellung eines essbaren proteinartigen Stoffes, geeignet zur Verwendung als Nahrungsmittel, umfassend das Fermentieren von Pilzzellen der Ordnung der Mucorales in wässriger Flüssigkeit. Die Flüssigkeit enthält eine assimilierbare Stickstoff(N)-Quelle sowie eine assimilierbare Kohlenstoff(C)-Quelle. Der RNA-Gehalt der Pilzzellen wird im Verfahren auf unter 4 Gewichtsprozent reduziert. Zum Binden kann Hühnereiweiß verwendet werden, welches jedoch allergene Reaktionen beim Konsumenten hervorrufen kann.

Des Weiteren gelten viele Ascomyten als Verderbniserreger mit teils pathogenem Potential. Beispielsweise produziert *Aspergillus flavus* ein karzinogenes Mykotoxin. Auch gelten viele Fusarien als Schadorganismen in der Landwirtschaft und sind überdies in der Lage, sehr tolerante Überdauerungsformen, d.h. Sporen, zu bilden, die in einer biotechnologischen Prozessführung zu Problemen führen könnten.

US2009/0148558 betrifft ein Verfahren zur Herstellung von Fleischersatzprodukten basierend auf Pilzmyzel, umfassend das Produzieren des Pilzmyzels, das Mischen des Myzels mit einem Protein-Komplement sowie einem Bindemittel und das Texturieren der Mischung in eine Proteinform durch Extrudieren der Mischung. Die Kultivierung des Pilzmyzels erfolgt in Flüssigkultur, das Zuckerrohrextrakt enthält. Als Bindemittel wird auch hier Hühnereiweiß eingesetzt.

Neben den oben beschriebenen verschiedenen Schwierigkeiten im Stand der Technik, hochwertige proteinreiche Fleischersatzprodukte herzustellen, ohne z.B. auf Allergene als Bindemittel zurückgreifen zu müssen, lassen die bekannten Verfahren zur Herstellung von Fleischersatzprodukten insbesondere die Notwendigkeit einer ausreichenden Vitaminzufuhr bei vegetarischer oder veganer Ernährung außer Acht. Dies betrifft insbesondere Vitamin D als auch Vitamin B12, die kaum in pflanzlicher Ernährung vorkommen.

Vitamin D ist nur in wenigen Lebensmitteln in höheren Mengen enthalten, z.B. Lebertran oder Fisch. Die Eigensynthese von Vitamin D ist abhängig von der Sonneneinstrahlung und insgesamt nur bedingt ausreichend zur Deckung des Vitaminbedarfs. Vitamin B12 ist hauptsächlich in tierischen Produkten zu finden, weshalb ein Mangel vor allem bei vegetarischer und veganer Ernährung auftritt.

Vitamin D kann aus Vorstufen, wie beispielsweise das in Pilzen vorkommende Ergosterol, durch Bestrahlung mit UV-Licht hergestellt werden. Weiterhin kann Vitamin B12 durch Fermentation von Mikroorganismen hergestellt werden. So beschreibt die DE 20 2010 016 402 U1 Vitamin D2 optimierte Pilze als funktionelles Lebensmittel oder als Zusatz für funktionelle Lebensmittel. Aus der EP2580316A2 ist *Lactobacillus reuteri* als Vitamin B12-Produzent bekannt. Mikroorganismen wie *L. reuteri* sind in der Lage, Hydroxycobalamin, eine natürliche Vitamin B12-Form, zu synthetisieren. Da Hydroxocobalamin jedoch eine instabile Verbindung ist, wird diese Form in der industriellen Nutzung mit Hilfe von Cyanid zu Cyanocobalamin umgewandelt. Der somit vorhandene Cyanidgehalt kann jedoch vor allem bei empfindlichen Menschen belastend wirken.

Die durch Fermentation produzierten Vitamine können aufgereinigt und als Supplemente, z.B. in Kapselform, eingenommen werden.

Es besteht jedoch weiterhin ein Bedarf an veganen oder vegetarischen Nahrungsmitteln, insbesondere Fleischersatzprodukten, bzw. Ausgangsstoffen zur Herstellung von solchen Nahrungsmitteln, die sowohl eine gute, zur Verarbeitung geeignete Textur besitzen, als auch mit solchen Vitaminen angereichert sind, die ansonsten durch eine fleischlose Ernährung nicht ausreichend zugeführt werden.

## 2. Kurze Beschreibung der Erfindung

Das beschriebene Problem wird durch das erfindungsgemäße Verfahren zur Herstellung eines vitamin- und proteinreichen Produkts gelöst.

Die vorliegende Erfindung betrifft zunächst ein Verfahren zur Herstellung eines vitamin- und proteinreichen Produkts, umfassend die Schritte:

a) Kultivieren zumindest einer Art aus der Abteilung der Basidiomyceten submers in einem Nährmedium enthaltend zumindest einen kohlenhydrathaltigen Agrarnebenstrom oder Lebensmittelnebenstrom, um ein erstes Kultivierungsprodukt zu erhalten, wobei das erste Kultivierungsprodukt Biomasse der zumindest einen Art aus der Abteilung der Basidiomyceten umfasst;

b) Zugabe zumindest einer Vitamin B12-produzierenden Art der Gattung *Propionibacterium* und/oder der Gattung *Lactobacillus* zu dem ersten Kultivierungsprodukt; und

c) Kultivieren der zumindest einen Art der Gattung *Propionibacterium* und/oder der zumindest einen Art der Gattung *Lactobacillus* in dem ersten Kultivierungsprodukt, um ein zweites Kultivierungsprodukt zu erhalten, wobei das zweite Kultivierungsprodukt das vitamin- und proteinreiche Produkt ist und wobei das zweite Kultivierungsprodukt Biomasse der zumindest einen Art aus der Abteilung der Basidiomyceten und Biomasse der zumindest einen Vitamin B12-produzierenden Art der Gattung *Propionibacterium* und/oder *Lactobacillus* umfasst.

Die Basis des Verfahrens zur Herstellung des Produkts bilden Proteine aus mehrzelligen Ständerpilzen (Basidiomyceten), die üblicherweise als Speisepilze konsumiert werden. Ein gefahrloser Konsum des hergestellten Produktes ist somit sichergestellt. Des Weiteren werden bei der vorliegenden Erfindung kohlenhydratreiche Agrarnebenströme oder Lebensmittelnebenströme als Substrate für die Pilzkultivierung verwendet, was eine Ressourcenschonung bzw. Reststoffverwertung ermöglicht. Zum Einsatz können Agrarnebenströme wie zum Beispiel Gemüse-, Obsttrester und Isomaltulosemelasse, ein Nebenprodukt der Zuckerherstellung (Markenname Palatinose), kommen. Diese in der Agrarindustrie anfallenden Reststoffe umfassen nur schwer hydrolysierbare Kohlenhydrate, die anderweitig kaum genutzt werden können. Pilze sind in der Lage, durch Nutzung exogener Cellulasen, die cellulosehaltigen Kohlenhydratfragmente zu spalten und als Kohlenstoffquelle zu verwenden. Die verwendeten Pilze bilden neben Protein auch Ballaststoffe, wie etwa Chitin. Diese Ballaststoffe, die somit im hergestellten Produkt vorhanden sind, haben eine positive Auswirkung auf die Darmflora, insbesondere durch ihre anti-oxidative, anti-hypertensive, anti-inflammatorische, anti-koagulate, anti-karzinogene, anti-mikrobielle, hypocholesterolemische und antidiabetische Wirkungen. Weiterhin führen sie zu einem längeren Sättigungsgefühl, da sie als natürlicher Quellstoff Wasser binden.

Die gemeinsame Kultivierung von Basidiomyceten und Bakterien bietet einen wesentlichen Vorteil gegenüber getrennten Fermentationsreaktionen, da nur ein Reaktor, d.h. Fermenter, für die Produktion benötigt wird. Somit wird der Fermentationsprozess deutlich robuster gegenüber Kontaminationen. Auch ist nur ein Ernteschritt erforderlich, falls das zweite Kultivierungsprodukt weiterverarbeitet werden soll. Die gemeinsame Kultivierung bedeutet zudem eine immense Kostensenkung, da nur ein Medium für beide Organismen verwendet wird, ein automatisierter Prozessablauf gelingt und damit Arbeitszeit eingespart werden.

Des Weiteren wurde im Rahmen der Erfindung festgestellt, dass die Form der gemeinsamen Kultivierung von Basidiomyceten und Bakterien in Schritt c) entscheidend für das Wachstum der Bakterienarten ist. Insbesondere konnte kein bzw. kaum Wachstum von Bakterien auf einem Nährmedium, das nach der Kultivierung in Schritt a) von den Basidiomycetenarten abgetrennt wurde, gemessen werden. Die Form der Co-Kultivierung ist somit entscheidend, um ein Produkt zu erhalten, das einerseits insbesondere aufgrund der Basidiomycetenbiomasse proteinreich und andererseits aufgrund der bakteriellen Vitaminbiosynthese mit Vitamin B12 angereichert ist.

In einer weiteren bevorzugten Ausführungsform wird das Verfahren in einem Kultivierungsgefäß durchgeführt. Wie oben bereits beschrieben, können die Bakterien somit direkt zu der Basidiomycetenkultur zugegeben werden und in dem gleichen Kultivierungsgefäß, beispielsweise einem Fermenter oder Reaktor, kultiviert werden. Insbesondere kann das Kultivierungsgefäß ein Volumen von mindestens 2 L, vorzugsweise mindestens 3 L, weiter vorzugsweise mindestens 4 L fassen.

In einer weiteren bevorzugten Ausführungsform wird das Verfahren ohne Ernten der zumindest einen Art aus der Abteilung der Basidiomyceten aus dem ersten Kultivierungsprodukt durchgeführt. Somit können die Bakterien direkt in das Kultivierungsgefäß umfassend die Pilzkultur zugegeben werden, was, wie oben beschrieben, die Prozessführung erleichtert und Kontaminationen vorbeugt.

In einer weiteren bevorzugten Ausführungsform ist die zumindest eine Art aus der Abteilung der Basidiomyceten ausgewählt aus einer Gruppe bestehend aus *Agrocybe aegerita, Pleurotus roseus, Lentinula edodes, Laetiporus sulphureus, Pleurotus sapidus, Stropharia rugosoannulata,* und/oder *Wolfiporia cocos.*

Diese Basidiomycetenarten haben sich als besonders geeignet zur Kultivierung und als für den späteren Konsumenten unbedenklich gezeigt. Die genannten Arten zeigen hohe Wachstumsraten auf Agrarnebenströmen bzw. Lebensmittelnebenströmen und erzielen somit hohe Biomasseproduktionswerte.

In einer weiteren bevorzugten Ausführungsform ist die zumindest eine Vitamin B12-produzierende Art aus der Gattung *Propionibacterium* aus den Arten *Propionibacterium freudenreichii sups. freudenreichii* und/oder *Propionibacterium freudenreichii sups. shermanii* ausgewählt. In einer weiteren bevorzugten Ausführungsform ist die zumindest eine Vitamin B12-produzierende Art aus der Gattung *Lactobacillus,* insbesondere *Lactobacillus reuteri.*

Diese Bakterienarten haben sich als besonders geeignet zur Kultivierung in dem Nährmedium, in dem zunächst die Basidiomyceten kultiviert werden, gezeigt. Auch weisen diese Bakterienarten hohe Vitamin B12-Syntheseraten auf, die sich vorteilhaft im zweiten Kultivierungsprodukt wiederfinden.

In einer weiteren bevorzugten Ausführungsform enthält das zweite Kultivierungsprodukt eine Gesamtbiomasse im Bereich von 10 bis 50 g/L, bevorzugt 15 bis 45 g/L, weiter bevorzugt 20 bis 40 g/L, gemessen an der Trockenmasse.

In einer weiteren bevorzugten Ausführungsform weist das erste Kultivierungsprodukt eine Gesamtbiomasse im Bereich von 5 bis 45 g/L, vorzugsweise 10 bis 40 g/L, insbesondere vorzugsweise 15 bis 35 g/L, gemessen an der Trockenmasse, auf.

Es hat sich herausgestellt, dass das Erzielen einer Biomasse der kultivierten Basidiomycetenarten in diesem Bereich vorteilhaft für das nachfolgende Kultivieren der Bakterienarten ist. Somit können die Bakterienarten unter den gegebenen Nährstoffkonzentrationen, die nach der Kultivierung der Basidiomyceten vorliegen, hohe Teilungsraten und hohe Vitamin B12-Syntheseraten erreichen. Insgesamt kann hierdurch ein Produkt hergestellt werden, welches insbesondere durch die vorhandene Basidiomycetenbiomasse reich an Protein ist sowie durch die Bakterienbiomasse ausreichend mit Vitamin B12 angereichert ist.

In einer weiteren bevorzugten Ausführungsform wird das Kultivieren in Schritt a) bei einer Temperatur von 20 bis 28 °C durchgeführt, bevorzugt 22 bis 26 °C, insbesondere bevorzugt bei ca. 24 °C. In einer weiteren Ausführungsform verläuft das Kultivieren in Schritt a) aerob. Weiterhin kann das Kultivieren in Schritt a) bei einer Belüftungsrate von 0,1 bis 0,5 Volumen Luft pro Volumen Kulturmedium pro min (vvm), bevorzugt 0,2 bis 0,4 vvm, erfolgen.

Insbesondere kann das Kultivieren in Schritt a) bei einer Temperatur von 20 bis 28 °C, bevorzugt 22 bis 26 °C, insbesondere bevorzugt bei ca. 24°C, sowie aerob bei einer Belüftungsrate von 0,1 bis 0,5 vvm, bevorzugt 0,2 bis 0,4 vvm, erfolgen.

Durch die ausgewählten Temperaturbereiche und/oder die ausgewählten Belüftungsratenbereiche kann sichergestellt werden, dass die Basidiomycetenarten hohe Biomassesyntheseraten aufweisen.

In einer weiteren Ausführungsform erfolgt das Kultivieren in Schritt a) im Wesentlichen unter Lichtausschluss, insbesondere unter Ausschluss von Tageslicht.

In einer weiteren Ausführungsform kann der zumindest eine kohlenhydrathaltige Agrarnebenstrom Cellulose enthalten.

Basidiomyceten sind in der Lage, Cellulose aufzuschließen, und wachsen daher sehr gut auf Cellulose-haltigen Agrarnebenströmen.

In einer weiteren bevorzugten Ausführungsform ist der zumindest eine kohlenhydrathaltige Agrarnebenstrom oder Lebensmittelstrom ausgewählt aus der Gruppe bestehend aus Apfeltrester, Aroniatrester, Spinattrester, Granatapfeltrester, Rübenmelasse, Isomaltulosemelasse, Sonnenblumenkerntrester, Zwiebeltrester, Biertreber, Traubentrester, Heu, und/oder Molke.

Insbesondere auf diesen Agrarnebenströmen bzw. Lebensmittelnebenströmen haben sich gute Wachstumsraten für die Basidiomycetenarten gezeigt.

In einer weiteren Ausführungsform kann das Nährmedium keinen Zuckerrohr enthalten.

Durch das enzymatische Aufschließen von Cellulose benötigen Basidiomyceten keine Disaccharide, wie im Zuckerrohr vorkommend, um eine ausreichende Wachstumsrate zu erreichen. Somit kann das Nährmedium ressourcenschonend gestaltet sein.

Alternativ kann das Nährmedium Glucose umfassen. Dies erlaubt das Erzielen hoher Wachstumsraten.

Weiterhin kann das Nährmedium 5,6-Dimethylbenzimidazol umfassen. 5,6-Dimethylbenzimidazol ist Bestandteil des Vitamin B12-Komplexes. Durch die Zugabe im Nährmedium kann eine hohe Ausbeute von Vitamin B12 im zweiten Kultivierungsprodukt erzielt werden.

Glucose und/oder 5,6-Dimethylbenzimidazol können insbesondere zusammen mit der zumindest einer Vitamin B12-produzierenden Art der Gattung *Propionibacterium* und/oder der Gattung *Lactobacillus* zu dem ersten Kultivierungsprodukt zugegeben werden.

In einer weiteren bevorzugten Ausführungsform enthält das in Schritt a) eingesetzte Nährmedium weiterhin: 5 bis 25 g/L Kohlenhydrate, bevorzugt 10 bis 20 g/L Kohlenhydrate, weiterhin bevorzugt 12 bis 17 g/L Kohlenhydrate.

In einer weiteren Ausführungsform wird die zumindest eine Art aus der Abteilung der Basidiomyceten vor dem Kultivieren in Schritt a) vorkultiviert, vorzugsweise in einem flüssigen Nährmedium, vorzugsweise umfassend 2 % Malzextraktmedium, weiterhin vorzugsweise unter Lichtausschluss und über einen Zeitraum von 1 bis 20 Tage. In einer weiteren Ausführungsform erfolgt die Vorkultivierung bei einer Temperatur von 20 bis 28 °C.

In einer weiteren bevorzugten Ausführungsform enthält das in Schritt a) eingesetzte Nährmedium weiterhin:

zumindest eine Stickstoffquelle;
zumindest eine Magnesiumquelle;
zumindest eine Kaliumquelle und/oder eine Phosphatquelle;
Spurenelemente, wobei die Spurenelemente Verbindungen von Eisen(II), Zink(II), Kupfer(II) und Mangan(II) umfassen.

Das Nährmedium kann auf einen pH-Wert von 5-7, vorzugsweise 5,5-6,5 eingestellt werden.

Als Kaliumquelle und/oder Phosphatquelle kann insbesondere Dikaliumhydrogenphosphat und/oder Kaliumdihydrogenphosphat verwendet werden.

Als Stickstoffquelle kann Ammoniumnitrat, L-Asparagin und/oder Hefeextrakt verwendet werden.

Als Magnesiumquelle kann insbesondere Magnesiumsulfat verwendet werden.

Besonders bevorzugt enthält das in Schritt a) eingesetzte Nährmedium: 5 bis 25 g/L Kohlenhydrate, bevorzugt 10 bis 20 g/L Kohlenhydrate, weiterhin bevorzugt 12 bis 17 g/L Kohlenhydrate, zumindest eine Stickstoffquelle, vorzugsweise ausgewählt aus L-Asparagin und/oder Ammoniumnitrat und/oder Hefeextrakt, insbesondere vorzugsweise Ammoniumnitrat oder Hefeextrakt, Spurenelemente umfassend Verbindungen von Eisen(II), Zink(II), Kupfer(II), Mangan(II), eine Kaliumquelle und/oder Phosphatquelle.

Dieses Nährmedium hat sich als besonders vorteilhaft erwiesen für die Kultivierung der Basidiomyceten in Schritt a) und die Kultivierung der Bakterienarten in Schritt c). Sowohl optimale Basidiomyceten-Biomassen als auch optimale Vitamin B12-Konzentrationen konnten durch die Verwendung eines solchen Nährmediums erzielt werden.

In einer weiteren Ausführungsform kann das Nährmedium kein Milchprotein enthalten.

Somit ist das durch das Verfahren hergestellte Produkt für vegan lebende Menschen geeignet. Außerdem ist das hergestellte Produkt koscher sowie halal.

Alternativ kann das Nährmedium Molke umfassen. Molke als Lebensmittelnebenstrom eignet sich insbesondere zur Steigerung der Gesamtbiomasse sowie des Vitamin B12-Gehalts im zweiten Kultivierungsprodukt.

In einer weiteren bevorzugten Ausführungsform wird in Schritt b) die zumindest eine Vitamin B12-produzierende Art der Gattung *Propionibacterium* und/oder der Gattung *Lactobacillus* so zugegeben, dass eine Gesamtkeimzahl aller zugegebenen Bakterienarten in einem Bereich von $10^4$ bis $10^{10}$ CFU/ml, vorzugsweise $10^5$ bis $10^9$ CFU/ml, in dem ersten Kultivierungsprodukt vorliegt.

In einer weiteren bevorzugten Ausführungsform wird das Kultivieren der zumindest einen Vitamin B12-produzierenden Art der Gattung *Propionibacterium* und/oder der der Gattung *Lactobacillus* in Schritt c) bis zum Erreichen einer Vitamin B12-Konzentration im Bereich von 1 bis 20 ng/ml Kultur, vorzugsweise 2 bis 15 ng/ml Kultur, insbesondere vorzugsweise 3 bis 10 ng/ml, durchgeführt.

In einer weiteren Ausführungsform wird das Kultivieren der zumindest einen Vitamin B12-produzierenden Art der Gattung *Propionibacterium* und/oder der der Gattung *Lactobacillus* in Schritt c) bei einer Temperatur von 25 bis 40°C, bevorzugt 28 bis 37°C durchgeführt.

In einer weiteren Ausführungsform wird das Kultivieren der zumindest einen Vitamin B12-produzierenden Art der Gattung *Propionibacterium* und/oder der Gattung *Lactobacillus* in Schritt c) bei einer Belüftungsrate von weniger als 0,2 vvm, bevorzugt weniger als 0,1 vvm, weiterhin bevorzugt weniger als 0,05 vvm durchgeführt.

In einer weiteren Ausführungsform wird das Kultivieren der zumindest einen Vitamin B12-produzierenden Art der Gattung *Propionibacterium* und/oder der der Gattung *Lactobacillus* in Schritt c) im Wesentlichen anaerob durchgeführt.

Die Vitamin B12-produzierenden Bakterien können hierbei aerotolerant oder anaerob sein. In einer weiteren Ausführungsform sind die Vitamin B12-produzierenden Bakterien nicht aerob und/oder microaerophil. Die Vitamin B12 Biosynthese verläuft dadurch insbesondere über den anaeroben Syntheseweg. Der anaerobe Syntheseweg erleichtert insgesamt die Prozessführung, da somit keine Sauerstoffbelüftung in der Kultur in Schritt c) benötigt wird.

Insbesondere kann das Kultivieren der zumindest einen eine Vitamin B12-produzierenden Art der Gattung *Propionibacterium* und/oder der der Gattung *Lactobacillus* in Schritt c) bei einer Temperatur von 25 bis 40°C, bevorzugt 28 bis 37°C, im Wesentlichen anaerob bei einer Belüftungsrate von weniger als 0,2 vvm, bevorzugt weniger als 0,1 vvm, weiterhin bevorzugt weniger als 0,05 vvm durchgeführt werden, besonders bevorzugt anaerob.

In einer weiteren bevorzugten Ausführungsform ist das zweite Kultivierungsprodukt das vitamin- und proteinreiche Produkt.

Somit kann die Kultur ohne weitere Verarbeitungsschritte direkt verwendet werden.

In einer hierzu alternativen, bevorzugten Ausführungsform umfasst das Verfahren weiterhin die Schritte:

d) Ernten der zumindest einen Art aus der Abteilung der Basidiomyceten und der zumindest einen Vitamin B12-produzierenden Art der Gattung *Propionibacterium* und/oder *Lactobacillus* aus dem zweiten Kultivierungsprodukt;

e) Trocknen der geernteten zumindest einen Art aus der Abteilung der Basidiomyceten und der zumindest einen Vitamin B12-produzierenden Art der Gattung *Propionibacterium* und/oder *Lactobacillus* zum Erhalten des vitamin- und proteinreichen Produkts.

In einer weiteren Ausführungsform erfolgt das Ernten im Schritt d) durch Filtern des zweiten Kultivierungsprodukts. Das Filtern kann insbesondere durch einen Filter mit Porengrößen im Bereich von 7 bis 12 µm erfolgen.

Beispielsweise kann zum Filtern ein Büchnertrichter verwendet werden.

In einer alternativen Ausführungsform erfolgt das Ernten im Schritt d) durch Zentrifugieren des zweiten Kultivierungsprodukts. Das Zentrifugieren kann insbesondere bei einer Zentrifugalbeschleunigung im Bereich von 2000 g bis 5000 g über einen Zeitrahmen von 5 bis 15 min erfolgen.

In einer weiteren Ausführungsform erfolgt das Trocknen im Schritt e) durch Lyophilisieren und/oder Walzen und/oder Sprühtrocknung, insbesondere durch Lyophilisieren.

In einer weiteren Ausführungsform liegt nach dem Schritt e) der Wassergehalt der getrockneten zumindest einen Art aus der Abteilung der Basidiomyceten und der zumindest einen Vitamin B12-produzierenden Art der Gattung *Propionibacterium* und/oder *Lactobacillus* bei 1 bis 60 Gewichtsprozent, vorzugsweise bei 5 bis 30 Gewichtsprozent.

In einer weiteren bevorzugten Ausführungsform umfasst das erfindungsgemäße Verfahren weiterhin den Schritt:

Bestrahlen der zumindest einen Art aus der Abteilung der Basidiomyceten zumindest teilweise mit einer UV-Lichtquelle, vorzugsweise mit einer Wellenlänge in einem Bereich von 250 bis 350 nm, wobei der Schritt des Bestrahlens während des gesamten Verfahrens durchgeführt werden kann.

Das Bestrahlen kann in den Schritten a) und/oder c) erfolgen. In einer weiteren bevorzugten Ausführungsform wird das Bestrahlen nach dem Trocknen im Schritt e), insbesondere nach dem Lyophilisieren, durchgeführt.

Die Bestrahlung nach dem Trocknen hat sich als besonders effizient herausgestellt. Auch muss nicht die gesamte getrocknete Biomasse bestrahlt werden, sondern die Bestrahlung nur eines Teils davon kann ausreichen, um die gewünschte Vitamin D2 Konzentration in der Gesamtbiomasse zu erreichen.

In einer weiteren Ausführungsform wird das Bestrahlen der zumindest einen Art aus der Abteilung der Basidiomyceten zumindest teilweise mit einer UV-Lichtquelle so ausgeführt, dass ein Gehalt von Vitamin D2 in einem Bereich von 0,1 bis 0,5 µg Vitamin D2 / g Trockenmasse der zumindest einen Art aus der Abteilung der Basidiomyceten erhalten wird.

Somit würde der Verzehr von etwa 10 bis 50 g des bestrahlten, getrockneten vitamin- und proteinreichen Produkts ausreichen, um den täglichen Vitamin D-Bedarf von 5 µm zu decken.

In einer weiteren bevorzugten Ausführungsform wird in Schritt b) weiterhin zumindest eine Glutaminase-aktiven Bakterienart ausgewählt aus der Gattung *Lactobacillus* zugegeben, wobei vorzugsweise *Lactobacillus rhamnosus* und/oder *Lactobacillus reuteri* zugegeben wird. Insbesondere kann die Glutaminase-aktive Bakterienart auch die Vitamin B12-produzierende Bakterienart sein.

Glutaminase-aktive Bakterien können die von den Basidiomyceten synthetisierte Aminosäure Glutamin effizient in Glutamat umsetzen. Somit kann dem hergestellten Produkt, welches als Nahrungsmittel, insbesondere als Fleischersatzprodukt verwendet werden kann, ein angenehmer Geschmack bereits in der Prozessführung verliehen werden.

Durch Verwendung einer einzelnen Bakterienart, die Vitamin B12 produziert und gleichzeitig Glutaminase-aktiv ist, kann das Verfahren besonders effizient gestaltet werden. Das zweite Kultivierungsprodukt ist somit sowohl mit Vitamin B12 angereichert und benötigt keine weiteren Verarbeitungsschritte, um geschmacklich aufgewertet zu werden.

In einer weiteren Ausführungsform wird nach dem Schritt e) zumindest eine Glutaminase-aktive Bakterienart ausgewählt aus der Gattung *Lactobacillus* auf dem protein- und vitaminreichen Produkt kultiviert, wobei vorzugsweise *Lactobacillus rhamnosus* und/oder *Lactobacillus reuteri* kultiviert wird.

In einer weiteren Ausführungsform wird nach dem Schritt e) das vitamin- und proteinreiche Produkt einer Wärmebehandlung unterzogen, vorzugsweise bei 40 bis 70°C für einen Zeitraum von 10 bis 60 Minuten, zum Erhalten eines vitamin- und proteinreichen, RNA-armen Produkts.

Durch eine Wärmebehandlung kann auf diese Weise der Gesamt-RNA-Gehalt des Produkts reduziert werden und somit ein gesundheitlicher Mehrwert erzielt werden.

Insbesondere kann so ein purinarmes Produkt bereitgestellt werden, welches im Rahmen einer purinarmen Diät konsumiert werden kann.

Die vorliegende Erfindung betrifft weiterhin ein vitamin- und proteinreiches Produkt, herstellbar nach dem Verfahren einer der vorhergehenden Ausführungsformen.

In einer weiteren Ausführungsform kann das vitamin- und proteinreiche Produkt kein Hühnereiweiß enthalten.

Hühnereiweiße werden häufig als Bindemittel eingesetzt, sind jedoch wegen der vorteilhaften Textur des Basidiomycetenproteinanteils nicht notwendig für die Verarbeitung zu Lebensmitteln. Somit ist das Produkt für Menschen mit Hühnereiweißunverträglichkeit geeignet.

In einer weiteren Ausführungsform ist die biologische Wertigkeit des vitamin- und proteinreiches Produkts zumindest 50, vorzugsweise zumindest 60, insbesondere vorzugsweise zumindest 70.

Die vorliegende Erfindung betrifft weiterhin ein Nahrungsmittel, vorzugweise ein Fleischersatzprodukt oder Tierfuttermittel, enthaltend das vitamin- und proteinreiche Produkt.

In einer weiteren Ausführungsform kann das Nahrungsmittel, welches das vitamin- und proteinreiche Produkt enthält, ausgewählt sein aus Fleischprodukten, beispielsweise Streichwurst, Salami, Schinken, Schnitzel, Gyros, Döner, Hackfleisch, Chicken-Nuggets, Bratspieße, und/oder Speck.

Es hat sich unter anderem gezeigt, dass das vitamin- und proteinreiche Produkt eine hohe Klebkraft aufweist. Somit ist das vitamin- und proteinreiche Produkt insbesondere vorteilhaft in Nahrungsmitteln aus unterschiedlichen Fleischsorten, z.B. Döner, einzusetzen, um die Konsistenz und Textur zu verbessern.

In einer weiteren Ausführungsform kann das Nahrungsmittel keine tierischen Proteine enthalten.

Insbesondere kann das Nahrungsmittel ein fleischloses Produkt, das in der Form von Streichwurst, Salami, Schinken, Schnitzel, Gyros, Döner, Hackfleisch, Chicken-Nuggets, Bratspieße, und/oder Speck vorliegt, sein.

In einer bevorzugten Ausführungsform kann das Nahrungsmittel eine vegane Bratwurst, veganer Brotbelag, vegane Wurst, vegane Nuggets, vegane Frikadellen, und/oder vegane Schnitzel sein.

Das Nahrungsmittel kann das vitamin- und proteinreiche Produkt in einer Konzentration von 0,1 bis 99,9 Gew.-%, vorzugsweise 0,3 bis 80 Gew.-%, bezogen auf das Gesamtgewicht des Nahrungsmittels, enthalten.

In einer weiteren Ausführungsform kann das Nahrungsmittel weiterhin pflanzliche Proteine enthalten, vorzugsweise in einer Konzentration von höchstens 80 Gew.-%, weiterhin vorzugsweise höchstens 65 Gew.-%, bezogen auf das Gesamtgewicht des Nahrungsmittels.

Weiterhin kann das Nahrungsmittel ein getreidehaltiges Erzeugnis und/oder ein Kartoffelerzeugnis sein. Insbesondere kann bei getreidehaltigen Erzeugnissen das vitamin- und proteinreiche Produkt als Ersatzstoff für Gluten eingesetzt werden.

Insbesondere kann das vitamin- und proteinreiche Produkt bei diesen Nahrungsmitteln als eine Proteinquelle eingesetzt werden. Des Weiteren kann das vitamin- und proteinreiche Produkt den Geschmack und die Textur von Nahrungsmitteln verbessern und Gluten substituieren.

Die vorliegende Erfindung betrifft weiterhin die Verwendung des Vitamin- und proteinreichen Produkts zur Herstellung von Nahrungsmitteln, vorzugweise Fleischersatzprodukten und/oder Tierfuttermitteln.

Die vorliegende Beschreibung betrifft weiterhin ein Nährmedium (nicht beansprucht), enthaltend:

zumindest einen kohlenhydrathaltigen Agrarnebenstrom oder Lebensmittelnebenstrom, vorzugsweise wobei der zumindest eine kohlenhydrathaltige Agrarnebenstrom oder Lebensmittelnebenstrom ausgewählt ist aus der Gruppe bestehend aus Apfeltrester, Aroniatrester, Spinattrester, Granatapfeltrester, Rübenmelasse, Isomaltulosemelasse, Sonnenblumenkerntrester, Zwiebeltrester, Biertreber, Traubentrester- und/oder Heu;
zumindest eine Stickstoffquelle;
zumindest eine Magnesiumquelle;
zumindest eine Kaliumquelle und/oder eine Phosphatquelle;
Spurenelemente, wobei die Spurenelemente Verbindungen von Eisen(II), Zink(II), Kupfer(II) und Mangan(II) umfassen.

Dieses Nährmedium hat sich als besonders geeignet zur sequentiellen Co-Kultivierung gezeigt. Insbesondere konnten hohe Biomassewerte, basierend auf den Basidiomyceten, und hohe Vitamin B12-Werte, basierend auf der Biosynthese in den verwendeten Bakterien erzielt werden.

Das Nährmedium kann auf einen pH-Wert von 5-7, vorzugsweise 5,5-6,5 eingestellt werden. Als Stickstoffquelle kann insbesondere L-Asparagin, Ammoniumnitrat und/oder Hefeextrakt verwendet werden. Als Kaliumquelle und/oder Phosphatquelle kann insbesondere Dikaliumhydrogenphosphat und/oder Kaliumdihydrogenphosphat verwendet werden. Als Magnesiumquelle kann insbesondere Magnesiumsulfat verwendet werden. Das Nährmedium kann weiterhin Glucose und/oder 5,6-Dimethylbenzimidazol umfassen.

Der Begriff "Agrarnebenstrom" bezieht sich auf den Nebenstrom, der bei der Verarbeitung von landwirtschaftlichen Ressourcen zu industriellen Hauptprodukten anfällt. Der Begriff "Lebensmittelnebenstrom" bezieht sich auf den Nebenstrom, der in der industriellen Lebensmittelindustrie anfällt.

Der Begriff "Vitamin D" wird für die Vitamin D-Gruppe der Secosteroide verwendet und umfasst unter anderem Prävitamin D3, Vitamin D2 (Ergocalciferol), Vitamin D3 (Cholecalciferol), soweit nicht weiter spezifiziert.

Der Begriff "Vitamin B12" wird für die Gruppe der Cobalamine verwendet und umfasst unter anderem Aquacobalamin, Hydroxocobalman, Methylcobalamin, Cyanocobalamin, Adenosylcobalamin.

Der Begriff "Kultivierung" bezieht sich auf das Wachstum und/oder die Vermehrung von Organismen, und somit die Steigerung ihrer Biomasse. Der Begriff "Fermentation" wird vorliegend synonym zum Begriff "Kultivierung" verwendet.

3. **Kurze Beschreibung der** Figuren

Fig. 1    zeigt das Bakterienwachstum einer Bakterienkultur bestehend aus P. *freudenreichii* subspezies *shermanii* und *P. freudenreichii* subspezies *freudenreichii* als n-fache Steigerung der anfänglich zugegebenen Menge in KBE/mL (a), sowie die Gesamtbiomasse inklusive *Pleurotus sapidus* (PSA) in g/100 ml ermittelt durch Auswaage der geernteten und getrockneten Biomasse.

Fig. 2    zeigt das Bakterienwachstum einer Bakterienkultur bestehend aus P. *freudenreichii* subspezies *shermanii* und *P. freudenreichii* subspezies *freudenreichii* als n-fache Steigerung der anfänglich zugegebenen Menge in KBE/mL (a), sowie die Gesamtbiomasse inklusive *Pleurotus sapidus* (PSA) in g/100 ml ermittelt durch Auswaage der geernteten und getrockneten Biomasse.

Fig. 3    zeigt die Gesamtbiomasse in g/100 ml von *Pleurotus sapidus* (PSA)-Kulturen oder von Co-Kulturen von PSA mit Vitamin B12 produzierenden Bakterien auf M1-Medium (a) und M2-Medium (b), ermittelt durch Auswaage der geernteten und getrockneten Biomasse.

Fig. 4    zeigt das Bakterienwachstum einer Bakterienkultur bestehend aus P. *shermanii* und *P.freudenreichii* als n-fache Steigerung der anfänglich zugegebenen Menge in KBE/mL (a), sowie die Gesamtbiomasse inklusive *Pleurotus sapidus* (PSA) in g/100 ml ermittelt durch Auswaage der geernteten und getrockneten Biomasse.

Fig. 5    zeigt das Wachstum von *P. freudenreichii* subspezies *shermanii* auf Optimalmedium und auf *Pleurotus sapidus* (PSA)-Kulturüberständen.

Fig. 6    zeigt das Wachstum von *L. coli, L. monocytogenes, S. enteritidis enteritis,* und *P. aeruginosa* auf Optimalmedien und auf *Pleurotus sapidus* (PSA)-Kulturüberständen.

Fig. 7    zeigt die Steigerung des Glutamatgehalts in *Pleurotus sapidus* (PSA)-Myzel durch die Kultivierung mit *L. reuteri.*

Fig. 8    zeigt eine Vitamin B12-Standardkurve des verwendeten ELISA-Assays zu Bestimmung von Vitamin B12 in den Co-Kulturen.

Fig. 9    zeigt die Quantifizierung des *Pleurotus sapidus* (PSA)-Anteils im Lyophilisat, kultiviert auf dem Agrarnebenstrom Apfeltrester, gemessen über den Ergosterolgehalt (schwarz unterlegt); die Quantifizierung des Proteingehalts erfolgte nach der Methode von Kjeldahl (grau unterlegt).

Fig. 10    zeigt die Mengenelemente von *Pleurotus sapidus* (PSA), kultiviert auf Isomaltulosemelasse (PM).

Fig. 11    zeigt die essentiellen Spurenelemente von *Pleurotus sapidus* (PSA), kultiviert auf Isomaltulosemelasse (PM).

Fig. 12    zeigt die Substratzusammensetzung der verschiedenen, zur Kultivierung verwendeten Agrarnebenströme.

Fig. 13    zeigt die Vitamin D2-Konzentration einer *Pleurotus sapidus* (PSA)-Kultur auf Apfeltrester nach Belichtung des Lyophilisats bzw. der flüssigen Submerskultur, die anschließend lyophilisiert wurde.

Fig. 14    zeigt den Gesamt-RNA-Gehalt in Prozent einer unbehandelten und erhitzten *Pleurotus sapidus* (PSA)-Kultur auf Isomaltulosemelasse.

Fig. 15    zeigt die Zusammensetzung von *Pleurotus sapidus* (PSA) auf Isomaltulosemelasse.

Fig. 16    zeigt die Quantifizierung der einzelnen Aminosäuren von *Pleurotus sapidus* (PSA) auf Isomaltulosemelasse.

Fig. 17    zeigt einen optischen Vergleich von pflanzlichen "Bratwürsten" fein (I bis VI) mit einer handelsüblichen Bratwurst auf Fleischbasis (VII) und mit pflanzlichen "Bratwürsten" grob (VIII bis XIII).

Fig. 18    zeigt einen erfindungsgemäßen Prozess zur Gewinnung eines protein- Vitamin B12- und Vitamin D-reichen sowie glutamathaltigen Produkts.

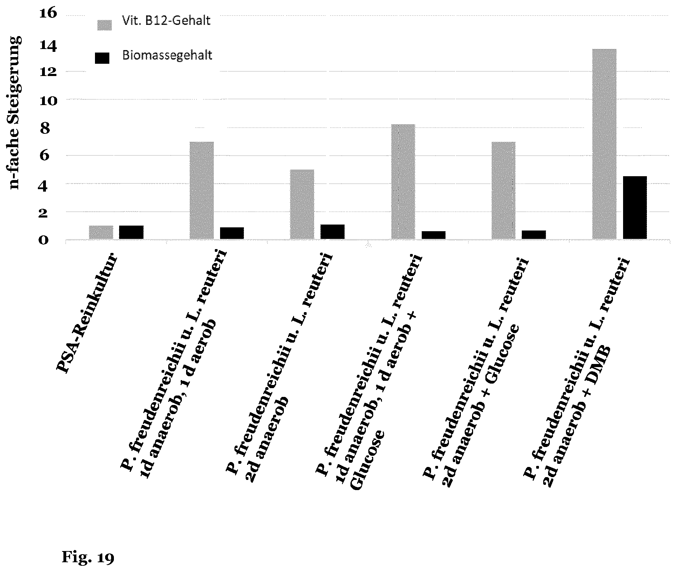

Fig. 19    zeigt die n-fache Steigerung der Biomasse und des Vitamin B12-Gehalts von Kulturen bestehend aus *Pleurotus sapidus* (PSA) sowie von Kulturen bestehend aus PSA, *P. freudenreichii* und *L. reuteri* in verschiedenen Kultivierungsbedingungen.

Fig. 20    zeigt die n-fache Steigerung der Biomasse und des Bakteriengehalts (in KBE/ml) unter Verwendung von Palatinose bzw. Molke.

Fig. 21    zeigt einen Versuchsreaktor nach 62 h Kultivierung einer PSA-Reinkultur.

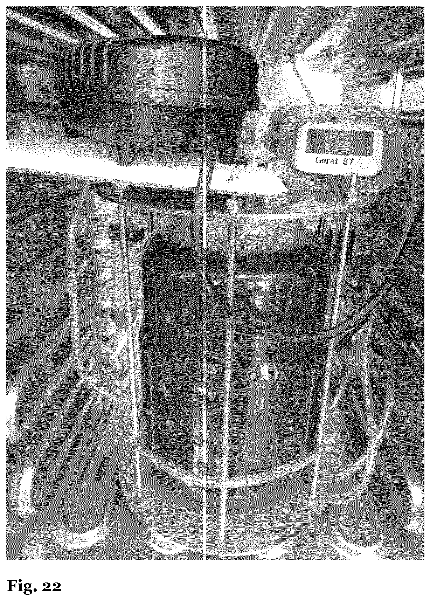

Fig. 22    zeigt einen Versuchsreaktor während Kultivierung einer PSA-Reinkultur.

## 4. Detaillierte Beschreibung der Erfindung

Die vorliegende Erfindung basiert auf der submersen Co-Kultivierung von Basidiomycetenarten und Vitamin B12 produzierenden Bakterienarten in einem Nährmedium. Das Nährmedium umfasst vorzugsweise Agrarnebenströme bzw. Lebensmittelnebenströme, die Monosacharide, Disaccharide und/oder Oligosaccharide enthalten und/oder celluloseoder stärkehaltig sein können. Vorzugsweise eingesetzt werden als Agrarnebenströme Isomaltulosemelasse aus der Herstellung von Isomaltulose, Karottentrester, Apfeltrester Granatapfeltrester, Spinattrester und/oder Rübenmelasse. Lebensmittelnebenströme können Biertreber, Traubentrester und/oder Molke umfassen. Vorzugsweise erfolgt die Kultivierung der Basidiomyceten aerob bei einer Temperatur zwischen 20 und 28 °C, bis genügend Biomasse zur Verfügung steht. Im Anschluss erfolgt die Kultivierung von Vitamin B12-produzierenden Bakterien, vorzugsweise aus den Gattungen *Lactobacillus* und/oder *Propionibakterium* in dem Nährmedium. Die eingesetzten Bakterienstämme sind vorzugsweise Mikroorganismen, die in der Lebensmittelherstellung bereits gefahrenlos genutzt werden und als GRAS-Organismen ("generally recognized as safe") gelten. Bei der Kultivierung der Bakterienarten wird die Temperatur auf eine für das Bakterienwachstum günstige Temperatur angehoben. Des Weiteren können anaerobe Bedingungen geschaffen werden.
Während der Kultivierung der Basidiomycetenarten, der Kultivierung der Bakterienarten in dem Nährmedium, während der Ernte, und/oder während der Lyophilisierung der geernteten Arten kann mittels UV-B Bestrahlung Vitamin D2 in den Basidiomyceten produziert werden.
Vitamin D wird vom menschlichen Körper bei Sonneneinstrahlung gebildet oder mit der Nahrung aufgenommen. Dabei kommt Vitamin D in unterschiedlichen Formen vor, von denen insbesondere Vitamin D2 (Ergocalciferol), dass in Pilzen vorkommt, und Vitamin D3 (Cholecalciferol), das nur in tierischen Nahrungsmitteln enthalten ist, besonders relevant sind. Beide Verbindungen werden in der Leber zu dem Pro-Hormon 25-Hydroxycholecalciferol bzw. 25-Hydroxyergocholecalciferol und nachfolgend in der Niere zu dem Vitamin D-Hormon $1\alpha$,25-Dihydroxycholecalciferol bzw. $1\alpha$, 25-Dihydroxyergocholecalciferol umgewandelt. Pfifferlinge beinhalten beispielsweise 2,1 $\mu$g/100g, Champignons 1,9 $\mu$g/100g Vitamin D2. Zudem besitzen Pilze einen hohen Anteil an Ergosterol, welches durch Bestrahlung mit UV-B Lichtquellen in Vitamin D2 umgewandelt wird. Somit kann durch die Bestrahlung während des erfindungsgemäßen Verfahrens, die eine Umwandlung von Ergosterol in Vitamin D2 bewirkt, ein erheblicher Mehrwert im hergestellten Produkt erzielt werden.
Die Produktion von Vitamin D2 kann insbesondere durch Bestrahlung sowohl in der Submerskultur als auch in der gefriergetrockneten Variante des Myzels erfolgen. Hierbei kann man flexibel den Zeitpunkt der Bestrahlung festlegen.
Im Anschluss an die Kultivierung von Pilzen und Bakterien erfolgt die Ernte der gewonnenen Fermentationsprodukte. Diese werden anschließend mit Trocknungstechnologien wie Lyophilisierung, Walzen oder Sprühtrocknung zu Pulvern verarbeitet. Der Wassergehalt im hergestellten Produkt liegt beispielsweise im Bereich von 1 bis 15 Gewichtsprozent. Das Fermentationsprodukt kann aber auch mit hohem Wassergehalt weiterverarbeitet werden.
Je nach verwendetem Agrarnebenstrom oder Lebensmittelnebenstrom erscheint das Pulver hell oder bräunlich. Abhängig von der späteren Applikation können unterschiedliche Kombinationen von Pilz und Agrarnebenstrom/ Lebensmittelnebenstrom verwendet werden. Das Proteinpulver hat einen neutralen, je nach verwendetem Ausgangssubstrat einen nussigen oder an Pilze erinnernden Geschmack.

Durch die Trocknung entsteht ein Produkt mit hohem Protein- und Ballaststoff- und gleichzeitig geringem Fettanteil. Bei der Analyse der technologischen Eigenschaften hat sich herausgestellt, dass die Wasserbindekapazität, Ölbindekapazität oder Emulgierbarkeit vergleichbar mit denen pflanzlicher Proteine sind. Weiterhin ist die Maillardreaktion vergleichbar mit Fleischprodukten. Zudem ist in dem Pilzprotein die Klebekraft gegenüber pflanzlichen Proteinen gesteigert. Ebenfalls sind Parameter wie Härte, Kaufähigkeit und Gummiartigkeit vergleichbar, oder, insbesondere was die Gummiartigkeit betrifft, sogar besser als bei pflanzlichen Proteinen. Hinsichtlich Sprungkraft und Klebrigkeit im erhitzten Zustand gibt es ebenfalls keinen Unterschied zu pflanzlichen Proteinen. Insgesamt besitzt das Pilzprotein durchweg bessere oder annähernd gleich gute technofunktionelle Eigenschaften wie pflanzliche Proteine. Die biologische Wertigkeit ist im Vergleich zu anderen Lebensmitteln überraschend hoch (z.B. *Pleurotus sapidus* kultiviert auf Isomaltulosemelasse: Wertigkeit von 73). Als Referenz mit einer Wertigkeit von 100 wurde hierbei Vollei verwendet.

Die Weiterverarbeitung des Produkts kann nach bekannten Rezepturen aus der Wurstherstellung erfolgen.

**Im Rahmen der vorliegenden Erfindung werden die folgenden Verfahren und Messverfahren verwendet:**

## 1. Submerskultivierung von Basidiomyceten

Die Submerskultivierung von Basidiomyceten kann mit diversen kohlenhydrathaltigen Agrarnebenströmen oder Lebensmittelnebenströmen durchgeführt werden, wie beispielsweise Melassen aus der Zuckerherstellung, cellulosehaltigen Produkten aus der Saftherstellung wie Karottentrester und/oder Apfeltrester oder Schalen bzw. Presskuchen aus der Ölherstellung wie Sonnenblumenkernschalen und/oder Sonnenblumenkerntrester bzw. allen weiteren cellulosehaltigen Agrarnebenströmen und/oder Lebensmittelnebenströmen.

Zur Kultivierung können die Basidiomyceten zunächst auf Malzextraktagar (beispielsweise 20 g/L Malzextrakt, 15 g/L Agar, Agar) angezogen werden. Hierzu werden die Agarplatten mit einem ca. 1 cm$^2$ großen, mit Myzel bewachsenen Agarstück beimpft, mit Parafilm verschlossen und im Brutschrank bei 24 °C, z.B. über 7 Tage, kultiviert. Die zu ca. 80 % bewachsenen Platten werden bei 4 °C gelagert und regelmäßig nach dem gleichen Verfahren überimpft.

Für die Erstellung einer Vorkultur wird ein 2 cm$^2$ großes, mit Basidiomyceten bewachsenes Malzextraktagarstück unter sterilen Bedingungen in 200 ml 2%-iges, steriles Malzextraktmedium gegeben (1 cm$^2$/100 ml). Die Kultur kann mit Hilfe eines Mixers homogenisiert werden; dies ist aber nicht zwingend notwendig. Die Inkubation, um Basidiomycetenwachstum zu erhalten, kann beispielweise bei 24 °C, schüttelnd (150 rpm) unter Ausschluss von Licht über 4-19 Tage erfolgen (siehe Tabelle 1).

**Tabelle 1:** Vorkulturzeiträume verschiedener Basidiomycetenarten in Tagen [d].

| Stamm | Vorkulturdauer [d] |
|---|---|
| AAE (*Agrocybe aegerita*) | 11 |
| LED (*Lentinula edodes*) | 13 |
| LSU (*Laetiporus sulphureus*) | 13 |
| PSA (*Pleurotus sapidus*) | 6 |
| PEO (*Pleurotus roseus*) | 6 |
| SRU (*Stropharia rugosoannulata*) | 7 |
| WCO (*Wolfiporia cocos*) | 7 |

Für die Basidiomyceten-Hauptkultur kann beispielsweise Minimalmedium M1 (4,5 g/L L-Asparagin Monohydrat; 2,4 g /L Ammoniumnitrat, 1,5 g/L Kaliumhydrogenphosphat, 0,5 g/L Magnesiumsulfat, 1 ml/L Spurenelementlösung ( 0,5 g/L Eisen (II)-sulfat-Heptahydrat, 0,5 g/L Zinksulfat-Heptahydrat, 0,002 g/L Kupfer(II)-sulfat-Pentahydrat, 0,002 g/L Mangan(II)-chlorid-Tetrahydrat)) mit einer definierten Menge an Substrat (siehe Tabelle 2) im Erlenmeyerkolben (mit Stopfen verschlossen) zusammengeführt werden und das erhaltene Medium auf einen pH-Wert von 6 eingestellt und im Autoklav 20 min bei 120°C sterilisiert werden.

**Tabelle 2:** Mengen der eingewogenen Agrarnebenstromsubstrate in Minimalmedium M1, um einen einheitlichen Kohlenhydratgehalt von 15 g/L zu erhalten.

| Agrarnebenstromsubstrat | Konzentration [g/L] |
|---|---|
| Frischer Apfeltrester (AT) | 112 |
| Apfeltrester (ATD) | 24,6 |

(fortgesetzt)

| Agrarnebenstromsubstrat | Konzentration [g/L] |
|---|---|
| Aroniatrester (ARO) | 27,1 |
| Lyophilisierter Blattspinat | 32,3 |
| Granatapfeltrester | 19,3 |
| Rübenmelasse | 27,2 |

Im Anschluss wird die Basidiomycetenvorkultur in die Mediummischung mit einer Endkonzentration von 10 % Pilzvorkultur gegeben. Die Kultivierung erfolgt über 7-14 Tage bei 24 °C, schüttelnd (150 rpm) unter Lichtausschluss im Inkubator. Nach vollständigem Bewachsen der Kulturen werden diese 10 min bei 3283 g zentrifugiert und das Myzel mit vollentsalztem Wasser (VE-Wasser) dreimal gewaschen. Im Falle von Isomaltulosemelasse als Restsubtrat kann insbesondere M2 Medium für die Kultivierung der Basidiomyceten verwendet werden. Die Zusammensetzung des M2-Mediums ist wie folgt: 3g/L Hefeextrakt, 1,5 g/L Kaliumdihydrogenphosphat, 0,5 g/L Magnesiumsulfat-Hydrat, 1,0 mL Spurenelementlösung. Pro 100 ml M2 Medium wird 10 ml Palatinose zugegeben. Der weitere Ablauf entspricht dem oben beschriebenen.

## 2. Kultivierung der Basidiomycetenarten im 7,5 L-Fermenter

Der Fermenter wird mit 5 L Medium und einer entsprechenden Menge Substrat befüllt, der pH mit 1 M Natronlauge auf 6,0 eingestellt und autoklaviert. Inokuliert wird mit 500 ml Basidiomyceten-Vorkultur. Die Einstellungen des Fermenters betragen: 150 rpm Rührerdrehzahl, 24°C Temperatur und 0,3 vvm Belüftungsrate. Die erhaltenen Fermentationsprodukte werden anschließend durch Lyophilisierung bei -70°C, 37 mbar Druck, bis zu einem spezifischen Wassergehalt von 8 bis 12 Gewichts-% verarbeitet.

## 3. Co-Kultivierung von Basidiomyceten und Vitamin B12 Produzenten sowie Glutaminaseaktiven Bakterien am Beispiel von *Pleurotus sapidus* (PSA) mit *Propionibacterium freudenreichii* und *Lactobacillus reuteri* kultiviert auf dem industriellen Nebenstrom Isomaltulosemelasse

Für die Co-Kultivierung von Basidiomycten und Bakterien wird zunächst eine Vorkultur von *Pleurotus sapidus* (PSA) angezogen. Dazu wird ein ca. 2 cm$^2$ großes mit PSA bewachsenes Malzextraktagarstück unter sterilen Bedingungen in 200 ml 2%-iges, steriles Malzextraktmedium gegeben. Die Kultur kann mit Hilfe eines Mixers (höchste Leistungsstufe) homogenisiert werden; dies ist aber nicht zwingend notwendig. Im Anschluss erfolgt eine Inkubation bei 24 °C, schüttelnd (150 rpm) unter Ausschluss von Licht über 7 Tage.

Nach erfolgreicher Anzucht der Vorkultur wird die Hauptkultur beimpft. Dazu werden 90 ml Minimalmedium M1 oder M2 mit 10 ml Isomaltulosemelasse im Erlenmeyerkolben (mit Stopfen verschlossen) zusammengeführt, das erhaltene Medium auf pH-Wert von 6 eingestellt und im Autoklav 20 min bei 120°C sterilisiert. Im Anschluss wird die Basidiomycetenvorkultur in die Mediummischung mit einer Endkonzentration von 10% Pilzvorkultur gegeben. Die Kultivierung erfolgt über 7 Tage bei 24 °C, schüttelnd (150 rpm) unter Lichtausschluss im Inkubator.

Parallel werden Vorkulturen von *Lactobacillus reuteri* (*L. reuteri* DSM20016) und *Propionibacterium freudenreichii* (*P. freudenreichii*) subsp. *freudenreichii* (DSM20271) und *shermanii* (DSM4902) erstellt. Dazu werden 10 µl eines üblichen Glycerolstocks der jeweiligen Bakterien in 10 ml des jeweiligen Optimalmediums versetzt (für *L. reuteri:* B12 Assay-Medium (Fertigmedium von Sigma Aldrich, St. Louis USA); für *P. freudenreichii:* Propionibakterium-Medium (5 g/l Caseinpepton, 10 g/l Hefeextrakt, 16,8 g/L DL-Natriumlactat; pH-Wert 6,7 +/-0,2) und über Nacht bei 30°C (*Propionibacterium*) bzw. 37°C (*L. reuteri*) unter anaeroben Bedingungen (Wheatonröhrchen) inkubiert. Die Vorkulturen werden auf eine Bakterienkonzentration von ca. 6*10$^9$ CFU/ml eingestellt, um eine Endkonzentration in der PSA-Kultur von etwa 6*10$^7$ / 100 ml zu erhalten. Die Anfangs-OD$_{595nm}$ in den Pilzkulturen mit Bakterien sollte zum Startpunkt etwa 0,3 betragen.

Zu der über 7 Tage aerob kultivierten PSA-Kultur wird 1 ml der Bakterienvorkultur (*L. reuteri* oder Mischung aus *P. freudenreichii subsp. shermanii* und *subsp. freudenreichii*) gegeben und die Kulturen unter anaeroben Bedingungen im Anaerobiertopf mit Gaspack bei 30°C (Kultur mit *P. freudenreichii*) bzw. 37°C (Kultur mit *L. reuteri*) für 2 Tage inkubiert. Im Anschluss erfolgt die Ernte des Proteinpellets wie im nächsten Abschnitt beschrieben. Als Negativkontrolle und zum Vergleich des Basidiomycetenwachstums ohne Bakterien wird eine PSA Kultur ohne Zugabe der Bakteriensuspension mitgeführt. Die Temperaturen der Bebrütungszeit werden hierbei beibehalten.

Das bakterielle Wachstum wurde erfasst durch Anwendung klassischer, mikrobiologischer Zählverfahren (*L. reuteri* auf MRS-Agar; *Propionibacterium* auf Propionibakteriumagar) und zusätzlich durch Bestimmung der OD bei 595 nm zum Zeitpunkt 0, d.h. unmittelbar nach Zugabe der Bakterien in die PSA-Hauptkultur sowie am Ende der Kultivierung bzw. vor der Ernte.

Die Gesamtbiomasse wurde nach Ernte durch Auswaage bestimmt. Zunächst wurde das Feuchtgewicht durch Auswaage erfasst, im Anschluss das Proteinpelett bei 80°C getrocknet und erneut das Gewicht bestimmt. Dazu wurde die gesamte Kultur in einen Büchnertrichter gegeben, welcher mit einem Filterpapier der Porengröße 7-12 μm ausgelegt wurde. Der Büchnertrichter wird zuvor auf einer Saugflasche befestigt, an die eine Vakuumpumpe angeschlossen ist. Mit Hilfe der Vakuumpumpe wird die gesamte Flüssigkeit im Myzel abgenutscht. Das Myzel auf dem Filter wurde in eine leere Petrischale gegeben, deren Leergewicht definiert war, und bei 80°C getrocknet. Nach vollständiger Trocknung wurde auf der Feinwaage die Gesamtbiomasse in g bestimmt.

## 4. Bestimmung des Vitamin B12- Gehaltes

Die Bestimmung des Vitamin B12-Gehalts in den unterschiedlichen Proben wird mittels eines ELISA Kits (Cloud-Clone Corp.) durchgeführt. Die im Kit enthaltene Mikrotiterplatte ist mit einem monoklonalen Antikörper beschichtet, welcher mit Cyanocobalamin (CNCbl) spezifisch agiert. Mit Biotin markiertes CNCbl fungiert als Kompetitor zum CNCbl aus den Proben und eingesetzten Standards. Beide konkurrieren um die Antikörper auf der Platte. Die Bindung findet während einer einstündigen Inkubationsphase statt, nach welcher nicht gebundene Konjugate abgewaschen werden. Nach mehreren Waschschritten wird eine mit Avidin verknüpfte Merrettichperoxidase (HRP) hinzugegeben. Das Avidin wird vom Biotin aus dem Kompetitor gebunden und die angeknüpfte HRP bildet nach einem weiteren Inkubationsschritt in Verbindung mit der Substratlösung des Kits einen Farbkomplex. Die Farbintensität in den wells wird mittels Messung der Optischen Dichte (OD) bei 450 nm ermittelt. Sie verhält sich anti-proportional zu der in der Probe vorhandenen CNCbl-Konzentration.

Natürlich vorkommende Cobalaminformen können mit Hilfe des Kits nicht nachgewiesen werden, sondern müssen zu Cyanocobalamin umgewandelt werden. Eine Quantifizierung der Cobalaminanteile findet durch den Einsatz einer Standardreihe zwischen 0 und 10.000 pg/mL Cobalamin statt, welche in den Untersuchungen mitgeführt wird. Die ermittelte OD der Standardproben wird zum Logarithmus (log) der Standardkonzentration aufgetragen. Es ergibt sich eine Gerade, durch deren Formel der Logarithmus der Proben-OD berechnet werden kann. Eine Umkehrung des Logarithmus liefert den Cobalamingehalt der Proben in pg/mL.

Die Probenvorbereitung zur Umwandlung sämtlicher Cobalaminformen in Cyanocobalamin wird wie folgt durchgeführt:

Die Co-Kulturen werden 10 min bei 6.000 rpm zentrifugiert, der Überstand verworfen, das Pellet einmal mit ddH2O gewaschen. Anschließend wird eine definierte Menge ddH2O zugegeben und mit Glaskugeln (Durchmesser 0,25-0,5 5 mm) im Verhältnis 1:2 gemischt. Der Zellaufschluss erfolgt mit Hilfe von Ultraschall unter Verwendung eines Sonikators (Sonifier 250 d Branson) für 10 min bei 60% Amplitude (1 min Puls, 1 min Pause). Die aufgeschlossenen Kulturen werden erneut bei 6000 rpm für 10 min zentrifugiert. Anschließend erfolgt die Umwandlung in Cyanocobalamin durch Zugabe von 10%-igem KCN mit einer Endkonzentration von 2% KCN in der jeweiligen Kultur gefolgt von einer 10-minütigen Inkubation bei Raumtemperatur und anschließender Lagerung auf Eis. Anschließend erfolgt die Durchführung des ELISAs gemäß Herstellerangaben (ELISA KIT for Cyanocobalamin Cloud-Clone Corp.). Als Kontrolle für die Umwandlung von Cobalamin zu Cyanocobalamin wird eine definierte Menge an reinem Hydroxycobalamin (5.000 pg/ml) mitgeführt.

## 5. Bestimmung der Umwandlung von Glutamin in Glutamat

Im Aminosäureanalyseprofil von PSA zeigen sich hohe Gehalte an Glutamin, die durch Nutzung von Glutaminaseaktiven Bakterien in Glutamat umgewandelt werden können. *Lactobacillus rhamnosus* und *Lactobacillus brevis* können beispielsweise als Glutaminase-aktive Bakterien verwendet werden. Überdies hat sich gezeigt, dass auch *Lactobacillus reuteri* eine deutliche Glutaminaseaktivität aufweist. Neben der reinen Bestätigung der Glutaminaseaktivität wurde im Anschluss analysiert, ob die Zugabe von *L. reuteri* zu gefriergetrocknetem, gemahlenem PSA-Myzel, kultiviert auf Isomaltulosemelasse, zur Umwandlung des im Pilzmyzel enthaltenen Glutamins zu Glutamat führt. Die Vorgehensweise der Probenvorbereitung sowie der Bestimmung der Umwandlung zu Glutamat ist wie folgt:

In Wheaton-Röhrchen wird 0,1 g gefriergetrocknetes, gemahlenes, hitzebehandeltes (30 min bei 150°C) PSA-Myzel (mit Isomaltulosemelasse als Substrat kultiviert) eingewogen und 300 μl 1M Natriumacetatpuffer (pH 5,8), 10 μl Alcalase 0,1 % (v/v) und 800 μl Flavourzyme 8 % (v/v) zugegeben und auf 10 ml mit H$_2$O aufgefüllt. Bei Alcalase und Flavourzyme handelt es sich um zwei Enzymmischungen, die verschiedene Endo- und Exoproteasen enthalten. Diese sollen die Proteine des Pilzmyzels spalten, so dass diese leichter von *L. reuteri* aufgenommen werden können. Zu einem dieser beiden Ansätze werden 5 μl einer *L. reuteri*-Übernachtkultur gegeben, der andere dient als Negativkontrolle. Die Inkubation der Ansätze erfolgt bei 37°C über Nacht. Im Anschluss werden die Kulturen 10 min bei 6.000 rpm zentrifugiert, der erhaltene Überstand wird verworfen und das Pellet mit 50mM Natriumphosphatpuffer mit 1mM PMSF-Endkonzentration gewaschen und 5 Minuten bei 6000 rpm zentrifugiert. Dieser Vorgang wird zweimal wiederholt. Dem erhaltenen Pellet wird 1 ml 50 mM Natriumphosphatpuffer zugegeben und die Zellen mit Hilfe von Glaskugeln im Verhältnis 1:2 w/v und Vortexer 10 Minuten aufgeschlossen. Abschließend wird 10 Minuten bei 6.000 rpm zentrifugiert und der erhaltene Überstand in ein 1,5 ml Reaktionsgefäß überführt. Die Proben werden auf Eis gelagert bis zur Analyse mittels einem Glutamat-Assay Kit (abcam, Cambridge, UK). Dieses misst freies Glutamat. Der enthaltene Enzym-Mix erkennt Glutamat als spezifisches Substrat, was zu einer proportionalen Farbentwicklung führt. Diese kann dann kolorimetrisch bei einer OD von 450 nm gemessen werden. Diese Messungen werden mit dem Plattenreader Infinite 200 Pro von Tecan durchgeführt.

## 6. Bestimmung des Vitamin D-Gehaltes

Durch Belichtung des Basidiomycten-Myzels mit UV-B Strahlung (Arimed B 12 UV-Lampen, JW Sales GmbH, Stuttgart) kann Ergosterol, das in der Zellmembran lokalisiert ist, in Vitamin D2 umgewandelt werden. Ergosterol und Vitamin D2 werden mittels HPLC-DAD identifiziert und quantifiziert (Absorptionsmaxima: Ergosterol 282 nm, Vitamin D2: 265 nm. Die Nachweis- und Bestimmungsgrenzen werden nach DIN 32645 (Kalibrierverfahren) mit n=7; Signifikanzniveau 99% und k=3 bestimmt. Die Nachweisgrenze beträgt 1,1 $\mu$g/ml, die Bestimmungsgrenze 4,0 $\mu$g/ml.

Folgendes System wird verwendet.
Säulen: Chromolith Performence Reserved/Phase-18 e 100-4,6 mm (Länge-Durchmesser) (mit Vorsäule) und EC 250/4 Nucleosil 100-5 C18, in Reihe geschaltet HPLC-DAD: La Chrom System L-7100/L-7200/D-7000/L-7455 von Merck Hitachi Eluenten: Methanol, HPLC-Grade (A), Acetonnitril HPLC- Grade (B) und 0,05%ige Ameisensäure (C)
Flussrate: 1 ml/min (Gradient)

| Gradient: Zeit (min) | % A | % B | % C |
|---|---|---|---|
| 0 | 0 | 70 | 30 |
| 2 | 0 | 100 | 0 |
| 10 | 0 | 100 | 0 |
| 20 | 5 | 95 | 0 |
| 50 | 0 | 100 | 0 |
| 55 | 5 | 95 | 0 |
| 60 | 0 | 70 | 30 |

Injektionsvolumen: 10 $\mu$l
Software: HPLC System Manager HSM Manager, Version 4.1
Standards: Ergocalciferol ($\leq$ 98% Sigma), Cholecalciferol (99,9%, Supelco), Ergosterol ($\geq$75,0 %, Sigma) und 7-Dehydrocholesterol ($\geq$ 95,0%, Sigma)

Die Quantifizierung von Vitamin D2 erfolgt über die Peakflächenverhältnisse Analyt zu internem Standard der Kalibriergerade und unter Berücksichtigung der Probenvorbereitung.

$$\text{Vitamin D2 } [\mu g(gTM)^{-1}] = (\text{Vitamin D2 } [\mu g\ ml^{-1}]^* \ V_{MeOH\ (ml)})/E[g])^*(100/(100-\% \text{ Feuchte})$$

VMeOH: Volumen an Methanol, in dem der Rückstand aufgenommen wurde [ml]
E: Probeneinwaage [g]
% Feuchte: mittels Feuchtebestimmer ermittelte Restfeuchte

Probenvorbereitung:

Das lyophilisierte Pilzmyzel wird mit flüssigem Stickstoff zermahlen, ca. 2 g in Braunglasrundkolben eingewogen und mit 50 ml Ethanol, 4 ml Natrium-Ascorbatlösung (17,5 g in 100 ml 1M Natronlauge), 10 ml KOH/H2= (50/50, w/w) und 0,5 ml internem Standard Vitamin D3 (200 $\mu$g/ml) während 1 h bei 80°C unter Rückfluss verseift. Nach Zugabe von 50 ml VE-Wasser, Abkühlen auf Raumtemperatur und Filtration wird mit 50 ml Diethylether, 50 ml n-Pentan/10 ml Ethanol, 50 ml n-Pentan, 20 ml n-Pentan extrahiert. Die organischen Phasen wurden vereint, drei Mal mit 50 ml 3% KOH in 5%igem Ethanol gewaschen und anschließend mit VE-Wasser neutral gewaschen, über Natriumsulfat getrocknet (über Nacht, 4°C), abfiltriert und das Lösungsmittel bis zur Trockene eingeengt (40 °C, Rotationsverdampfer). Der Rückstand wurde in 1,5 ml Methanol aufgenommen, im Ultraschallbad 5 min gelöst und abzentrifugiert (10 min, 4 °C, 18.000 g). Nach Membranfiltration (0,22 $\mu$m) wurde die Lösung zur Quantifizierung mittels HPLC eingesetzt.

## 7. Aminosäureanalytik

Die Identifizierung und Quantifizierung der Aminosäuren im lyophilisierten Pilzmyzel erfolgte mittels Aminosäureanalysator. Hierbei wurden die Proteine zunächst einer Totalhydrolyse unterworfen.

- Aminosäureanalysator S 433 für Protein-Hydrolysate von Sykam
- Säulen: Trennsäule LCA K13 S/N, Filtersäule LCA K04 S/N
- Eluenten: Natrium-Citrat Pufferlösung, pH 3,4 (A), Natrium-Citrat Pufferlösung, pH 10,85 (B),
- Regenerierlösung (RegSol Na)
- Reagenz: Ninhydrin, pH 10,85
- Waschlösung: Ethanol/Isopropanol/Wasser (250/250/500 v/v/v)
- Flussrate: 0,45 mL min-1 (Gradient)
- Gradient:

| Zeit [min] | A [%] | B [%] | RegSol Na [%] |
|---|---|---|---|
| 0 | 100 | 0 | 0 |
| 5 | 100 | 0 | 0 |
| 11 | 95 | 5 | 0 |
| 13 | 80 | 20 | 0 |
| 25 | 70 | 30 | 0 |
| 29 | 30 | 70 | 0 |
| 31 | 20 | 80 | 0 |
| 33 | 10 | 90 | 0 |
| 41 | 0 | 100 | 0 |
| 49 | 0 | 0 | 100 |
| 49,1 | 0 | 0 | 100 |
| 52 | 0 | 0 | 100 |
| 52,1 | 100 | 0 | 0 |

- Injektionsvolumen: 50 μL
- Software: Chromstar, Version 7
- Standards: Aminosäure-Kalibrier-Lösung (Gemisch aus Aminosäuren mit bekannten Konzentrationen für Hydrolysate) (Sykam), L-Tryptophan ($\geq$ 99,0%, Roth).

Für die Quantifizierung der Aminosäuren wurde eine Einpunkt-Kalibrierung durchgeführt.

Die Berechnungen werden wie folgt durchgeführt:

$$m_{(Faktor)} \, [\text{mg TM}] = \frac{E-[TM-(TM*\%Feuchte/100])}{V_{(HCl)}} * V$$

$$w_{(AS)} \, [\text{g (100g TM)}^{-1}] = \frac{c_{(AS)}[mol] * M_{(AS)}[g \; mol^{-1}]}{m_{(Faktor)}[g]} * 100$$

mit

$m_{(Faktor)}$    Umrechnungsfaktor von nmol mL$^{-1}$ zu nmol mg$^{-1}$

TM    Trockenmasse mit Restfeuchte in mg

%Feuchte    Mittels Feuchtebestimmer ermittelte Restfeuchte [%]

E    Einwaage [mg]

$V_{(HCl)}$    Volumen an eingesetzter 6 M Salzsäure (3.4.10.1)

$V_{(Aliquot)}$    Volumen an eingedampftem Aliquot

$W_{(AS]}$    Massenanteil jeder einzelnen Aminosäure in g (100g TM)$^{-1}$

$C_{(AS)}$    Stoffmengenkonzentration einer Aminosäure in mol mL$^{-1}$

$M_{(AS)}$    Molmasse der entsprechenden Aminosäure

Die Probenvorbereitung für die Aminosäureanalytik erfolgt wie nachfolgend erläutert.

Saure Hydrolyse zur Gesamtaminosäurebestimmung:

Das lyophilisierte Pilzmyzel wird gemörsert, ca. 250 mg in Pyrexröhrchen eingewogen und mit 6 mL 6 M HCl (0,1% Phenol) versetzt. Zur Vermeidung von Oxidationen wird der Sauerstoff durch Einleiten von Stickstoff entfernt. Hydrolysiert wird für 24 und 48 h bei 110 °C im Trockenschrank. Nach Abkühlen auf Eis wird abzentrifugiert (20 min, 4 °C, 3.283 g) und membranfiltriert (0,22 $\mu$m). Zur Abtrennung der Säure wird ein Aliquot (200 $\mu$L) bei 130 °C bis zur Trockene eingedampft und in 1 mL Probenverdünnungspuffer (pH 2,20) aufgenommen. Nach Verdünnen mit Probenverdünnungspuffer (1:5) wird die Lösung zur Quantifizierung mittels Aminosäureanalysator eingesetzt.

Basische Hydrolyse zur Bestimmung von Tryptophan:

Das lyophilisierte Pilzmyzel wird gemörsert, ca. 250 mg in Pyrexröhrchen eingewogen und mit 6 mL 5 M NaOH (0,1% Phenol) versetzt. Zur Vermeidung von Oxidationen wird der Sauerstoff durch Einleiten von Stickstoff entfernt. Hydrolysiert wird für 24 und 48 h bei 110 °C im Trockenschrank. Nach Abkühlen auf Eis wird abzentrifugiert (20 min, 4 °C, 3.283 g) und membranfiltriert (0,22 $\mu$m). Ein Aliquot (200 $\mu$L) wird bei 130 °C bis zur Trockene eingedampft und in 1 mL Probenverdünnungspuffer (pH 2,20) aufgenommen. Nach Verdünnen mit Probenverdünnungspuffer (1:5) wird die Lösung zur Quantifizierung mittels Aminosäureanalysator eingesetzt.

Oxidation vor der Hydrolyse zur Bestimmung von Cystein und Methionin:

Das lyophilisierte Pilzmyzel wird gemörsert, ca. 250 mg Myzel in Pyrexröhrchen eingewogen und mit 5 mL 5 M Oxidationslösung (30 %iges H2O2 in 98 %iger Ameisensäure und 0,1 % Phenol) versetzt. Die Pyrexröhrchen werden verschlossen und für 16 h bei 0 °C im Eisbad inkubiert. Durch Zugabe von Natriumdisulfit wurde die Oxidation abgebrochen und 5 mL 6 M HCl (0,1 % Phenol) zugefügt. Die Hydrolyse erfolgt für 24 h bei 110 °C im Trockenschrank. Nach Abkühlen auf Eis wird abzentrifugiert (20 min, 4 °C, 3.283 g) und membranfiltriert (0,22 $\mu$m). Der pH-Wert wird mittels 1 M Natriumhydroxid auf 2,20 eingestellt. 200 $\mu$L werden zum Eindampfen entnommen und der Rückstand in 1 mL Probenverdünnungspuffer (pH 2,20) aufgenommen. Nach Verdünnen mit Probenverdünnungspuffer (1:5) wird die Lösung zur Quantifizierung mittels Aminosäureanalysator eingesetzt.

## 8. Biologische Wertigkeit

Die Biologische Wertigkeit (BW) ist die bekannteste Methode zur Abschätzung der Qualität von Proteinen in Lebensmitteln. Sie gilt als Maß dafür, wie viel eines aufgenommenen Nahrungsproteins in körpereigenes Protein umgewandelt werden kann.

Die biologische Wertigkeit ergibt sich aus folgender Gleichung:

$$BW = (\text{retinierter Stickstoff/absorbierter Stickstoff}) * 100$$

Je höher die biologische Wertigkeit der aufgenommenen Proteine ist, desto weniger Protein muss zugesetzt werden, um eine ausgeglichene Protein- und Stickstoffbilanz zu erreichen. Als wichtigstes Kriterium für die biologische Wertigkeit gilt die Zusammensetzung der Aminosäuren in einem Lebensmittel. Je mehr proteinogene Aminosäuren darin enthalten sind und je höher der Gehalt an essentiellen Aminosäuren ausfällt, desto höherwertig wird das Protein eingestuft.

Tierische Proteine besitzen in der Regel eine höhere biologische Wertigkeit als pflanzliche Proteine. Als "Referenzprotein" zur Qualitätsbewertung anderer Nahrungseiweiße wurde Hühnervollei ausgewählt, dem eine biologische Wertigkeit von 100 bzw. 1.0 zugeordnet wird. Die Angaben zur biologischen Wertigkeit aller anderen Proteine erfolgen somit im Vergleich zu Vollei. Der Referenzwert "100" des Volleis entspricht jedoch keiner 100-prozentigen Umsetzung von diesem, wodurch für die biologische Wertigkeit ein Wert von 100 gerade von kombinierten Lebensmitteln leicht übertroffen werden kann.

Durch geschickte Lebensmittelkombinationen kann die biologische Wertigkeit erheblich gesteigert werden, da sich die Aminosäuren verschiedener Lebensmittel gegenseitig ergänzen und Defizite ausgeglichen werden können (Ergänzungswert). Die Kombination von Nahrungsproteinen spielt besonders in solchen Ländern eine wichtige Rolle, in denen die Ernährung nur wenige tierische Lebensmittel vorsieht.

Mittels nachfolgender Berechnung wird die Biologische Wertigkeit berechnet:

$$EAAi: \quad \sqrt[n]{\left(\frac{Ile_T}{Ile_R} * 100\right) * \left(\frac{Leu_T}{Leu_R} * 100\right) * \left(\frac{Lys_T}{Lys_R} * 100\right) \dots}$$

EAAi: Essential Amino Acid Index (Index der essentiellen Aminosäuren)
BW= 1,09*EAAi-11,7

### 9. Bestimmung von Mineralstoffen/Zuckern

Die Proben werden mit Königswasser im Mikrowellensystem aufgeschlossen. Im Anschluss wird eine ICP-MS (Inductively coupled plasma mass spectrometry) durchgeführt. Durch einen hochfrequenten Strom wird ein Argon-Plasma induziert und die Probe auf 5.000 - 10.000 °C erhitzt. Die im Plasma generierten Ionen werden in Richtung des Analysators des Massenspektrometers durch ein elektrisches Feld beschleunigt und damit wird die Erfassung von Elementen und deren Isotope ermöglicht.
Die Bestimmung von Glucose und Fructose sowie die Erfassung der Umsetzung von D-Glucose und D-Fructose aus dem Substrat erfolgt enzymatisch.

### 10. Bestimmung des Gesamtstickstoffgehaltes nach Kjeldahl (Rohprotein)

Der Gesamtstickstoffgehalt wurde nach Kjeldahl (Kjeldahl 1883, modifiziert nach Matissek et al. 2010) jeweils in Doppelbestimmung quantifiziert. Die Proben wurden in stickstofffreie Pergamentschiffchen eingewogen und mit einer Glasperle, einer halben Katalysatortablette und 15 mL konzentrierter Schwefelsäure im Aufschlusskolben bei 400 °C für mind. 3 h aufgeschlossen, bis die Lösung eine grünliche Farbe angenommen hatte. Anschließend wurde eine Wasserdampfdestillation durchgeführt. Dazu wurden Natronlauge und einige Tropfen Sher-Indikator in den Aufschlusskolben gegeben. Der gebildete Ammoniak wurde in eine borsäurehaltige, mit Sher-Indikator versetzte Lösung übergetrieben. Die Titration erfolgte mit 0,1 M Salzsäure-Maßlösung.
Der Rohproteingehalt wurde nach der folgenden Gleichung berechnet.

$$P[\%TM] = \frac{a * 1,4008 * F}{E * 10} * \frac{100}{100 - \%Feuchte}$$

| | |
|---|---|
| P | Rohproteingehalt [% TM] |
| a | Verbrauch an 0,1 M HCl-Maßlösung [mL] |
| 1,40081 mL | 0,1 M HCl entspricht 1,4008 mg Stickstoff |
| F | Umrechnungsfaktor zur Errechnung des Rohproteingehalts (allgem. Faktor für Pilze: 4,38, für PSA: 4,97, für LED: 4,5) |
| E | Probeneinwaage [g] |
| 10 | Umrechnungsfaktor auf 100 g Probe und mg Stickstoff in g |
| %Feuchte | Mittels Feuchtebestimmer ermittelte Restfeuchte [%] |

### 11. Bestimmung des Aschegehaltes

Nach vollständiger Veraschung bei 550 °C wurde der Aschegehalt durch Differenzwägung der Quarztiegel ermittelt. Die Berechnung erfolgte nach folgender Gleichung:

$$\text{Asche } [\%TM] = \frac{\Delta Tiegel * 100}{E} * \frac{100}{100 - \%Feuchte}$$

| | |
|---|---|
| ΔTiegel | Differenz des Tiegels vor und nach Veraschung |
| Asche | Aschegehalt [% TM] |
| E | Probeneinwaage [g] |

%Feuchte    Mittels Feuchtebestimmer ermittelte Restfeuchte [%]

**12. Bestimmung des Gesamtkohlenhydratgehalts**

Der Gesamtkohlenhydratgehalt der Substrate wurde mittels Orcinol-Schwefelsäure-Assay bestimmt. Dazu wurden 10 mg Probe in 2 mL 2 M HCl hydrolysiert (2 h, 100 °C, 700 rpm) und anschließend membranfiltriert. Nach 1:50-Verdünnung mit Reinstwasser wurden 200 µL Hydrolysat (bzw. Standard) mit 800 µL Reagenzlösung (2 g L-1 Orcinol in konz. Schwefelsäure) versetzt, geschüttelt und 15 min bei 80 °C erwärmt. Nach Abkühlen auf Raumtemperatur wurde der Gehalt an Gesamtkohlenhydraten bei 420 nm photometrisch gegen Wasser bestimmt. Kalibriert wurde mit Glucose (10 - 100 µg mL-1).

**13. Bestimmung des Pilzanteils im Lyophilisat über Ergosterol**

Bei der Kultivierung von Pilzen auf Restströmen können Substratbestandteile vorhanden sein, die nicht oder nicht vollständig vom Pilz abgebaut werden und daher noch im geernteten Myzel vorhanden sind. Der Anteil an Pilz in diesem Myzel-Substrat-Gemisch kann über den Ergosterolgehalt bestimmt werden, da Ergosterol ausschließlich in Pilzen vorkommt. Zur Erstellung einer Kalibriergerade wurde der entsprechende Pilz in Malzextraktmedium kultiviert, das ausschließlich lösliche Bestandteile enthält und die Biomasse nach der Kultivierung daher zu 100% aus Pilzmyzel besteht. Dieses wurde für die Kalibrierung verwendet, bei der das Peakflächenverhältnis von Ergosterol zu 7-DHC (IST) gegen die Masse an Pilzmyzel [g TM] aufgetragen wurde.

Die Probenvorbereitung erfolgte wie bei der Probenvorbereitung im Rahmen der Vitamin D Analytik. Als interner Standard wurde 1 mL 7-Dehydrocholesterol (7-DHC, 1 mg mL-1) verwendet. Das Absorptionsmaximum von Ergosterol und 7-DHC liegt bei 282 nm. Bei dieser Wellenlänge wurde anschließend quantifiziert.

**14. Behandlung des Pilzmycels zur Reduktion des RNA-Anteils**

Das Pilzmycel wurde einer Temperaturbehandlung unterzogen. Dabei wurden Temperaturbereiche von 40-70°C sowie Inkubationszeiträume von 0- 40 Minuten getestet. Vor und nach der Behandlung wird der Rohproteingehalt bestimmt und im Anschluss die RNA mittels RNeasy® Plant Mini Kit extrahiert. Die Quantifizierung der RNA-Konzentration vor und nach der Hitzebehandlung erfolgte mittels Kapillargelelektrophorese.

**15. Technofunktionelle Untersuchungen**

Wasserbindekapazität/ Ölbindekapazität:

Eine wichtige Kenngröße für das Wasserbindevermögen ist die Wasserbindekapazität (WBK). Sie gibt die Masse an Wasser an, die von einem Gramm des Produkts gebunden werden kann. Zur Bestimmung dieser Kenngröße wird eine definierte Masse des Probenmaterials mit Wasser gesättigt, wobei das Wasser schrittweise bis zum Erreichen des Sättigungspunkts zugegeben wird. Es wird gerade so viel Wasser zugegeben, dass sich bei der Zentrifugation des Probenmaterials nur ein kleiner wässriger Überstand bildet. Der Vorteil der Zugabe eines Wasserüberschusses gegenüber der schrittweisen Zugabe besteht dabei in einem verminderten Arbeitsaufwand. Diesem steht allerdings der Nachteil gegenüber, dass mit dem überschüssigen Wasser auch lösliche Bestandteile des Probenmaterials abgetrennt werden. Dadurch kann es zu einer signifikanten Verfälschung des Messergebnisses kommen. Aus diesem Grund wurde sich dafür entschieden das Wasser schrittweise zuzugeben.

Die Ölbindekapazität (ÖBK) ist analog zur Wasserbindekapazität zu betrachten.

**Ausführungsbeispiele**

Die vorliegende Erfindung wird im Folgenden anhand verschiedener beispielhafter Verfahren und Produktbeispiele, die jedoch nicht als einschränkend anzusehen sind, illustriert.

**Beispiel 1: Screening unterschiedlicher Pilz-Substrat-Kombinationen**

Als Auswahlkriterium wurden das Wachstum [g TM L$^{-1}$], die Kultivierungsdauer und die Proteinausbeute [g L$^{-1}$], gemessen nach der Rohproteinbestimmungsmethode nach Kjendahl (s. unter 10. *"Bestimmung des Gesamtstickstoffgehalts nach Kjendahl (Rohprotein)"*), herangezogen.

Als gut geeignete Substrate für die Produktion von Basidiomyceten-Biomasse erwiesen sich unter anderem frischer Apfeltrester (Fischer) und Apfeltrester (Döhler) in Kombination mit *Pleurotus sapidus* (PSA) (Kulturdauer: 4 Tage, ca. 14 g TM L$^{-1}$, ca. 3 g L$^{-1}$ Protein). Des Weiteren erwiesen sich auch die Isomaltulosemelasse in Kombination mit PSA (Kulturdauer: 4 Tage, ca. 11 g TM L$^{-1}$, ca. 2,8 g L$^{-1}$ Protein), Zwiebeltrester in Kombination mit PSA (Kulturdauer: 13 Tage, ca. 34,5 g TM L$^{-1}$, ca. 3 g L$^{-1}$ Protein) und Karottentrester in Kombination mit *Lentinula edodes* (LED) (Kulturdauer: 6 Tage, ca. 9 g TM L$^{-1}$, ca. 2,3 g L$^{-1}$ Protein) als vielversprechend.

**Beispiel 2: Co-Kultivierung von *Pleurotus sapidus* und *Propionibacterium freudenreichii***

Zunächst wurde getestet, welche Temperaturbereiche die verwendeten Organismen tolerieren. Dabei wurde festgestellt, dass Basidiomyceten nur bis ca. 27 °C ein ausreichendes Wachstum zeigen und nach diesem Temperaturanstieg selbst bei Optimaltemperatur kaum noch Biomasse ausbilden. Die verwendeten Bakterien benötigen für ein ausreichendes Wachstum höhere Temperaturen, insbesondere liegt die Optimaltemperatur für Propionibakterien bei 30°C.

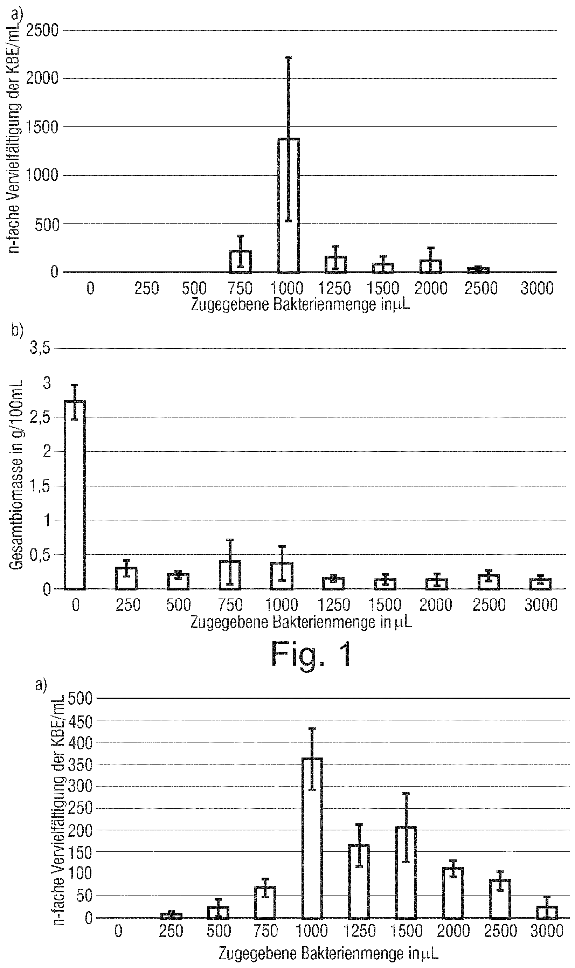

Zunächst wurde *Pleurotus sapidus* (PSA) in Minimalmedium M1, versetzt mit 10% (v/v) Isomaltulosemelasse, über 24 h aerob kultiviert (10 ml PSA Vorkultur und 90 ml M1/Isomaltulosemischung) und im Anschluss unterschiedliche Mengen einer Bakterienvorkultur mit ca. 6*10$^9$ CFU/ml (*P. freudenreichii subs. freudenreichii* und *subs. shermanii*) zugegeben. Es erfolgte eine Inkubation über 7 Tage unter Wechsel zu anaeroben Bedingungen bei 30°C. Festzustellen war ein Bakterienwachstum am Ende der Inkubationszeit insbesondere unter Verwendung von 1 ml Bakterienvorkultur. Allerdings war insgesamt nur sehr wenig PSA-Wachstum/Gesamtbiomasse erkennbar (siehe Fig. 1).

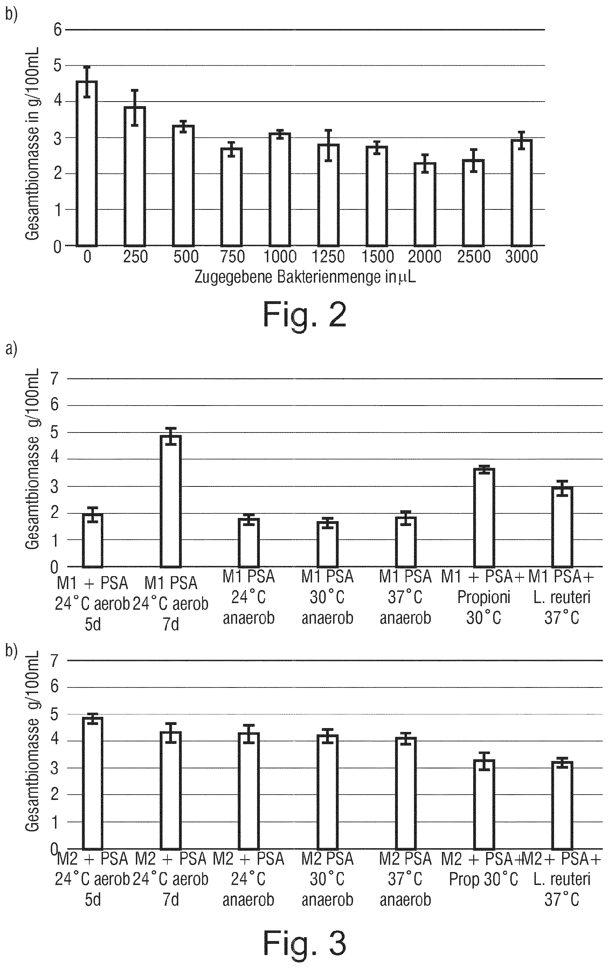

In weiteren Versuchen wurden 10 ml PSA Vorkultur in M1-Medium mit 10% Isomaltulosemelasse über 7 Tage schüttelnd (150 rpm) bei 24°C aerob inkubiert. Danach erfolgte die Zugabe von unterschiedlichen Mengen an Bakterienvorkultur (ca. 6 $^*$ 10$^9$ CFU/ml) bestehend aus *P. shermanii* und *P. freudenreichii* sowie die Umstellung auf anaerobe Bedingungen und ein Temperaturshift auf 30°C und weitere Inkubation für 48 h. Diese Variante führte bei Zugabe von 1 ml Bakterienvorkultur zu dem höchsten Bakterienwachstum und zufriedenstellendem Pilzwachstum (optisch erfasst) und ausreichender Gesamtbiomasse. Die Kontrollreaktion mit O $\mu$l zugegebener Bakterienvorkultur und Umstellung der Temperatur auf 30°C erzielte eine Gesamtbiomasse, d.h. Basidiomycetenbiomasse, von ca. 46 g/L, gemessen an der Trockenmasse (siehe Fig. 2).

In einer weiteren Versuchsreihe wurden die Gesamtbiomassen von Kulturen von PSA sowie Co-Kulturen von PSA und Vitamin B12 produzierenden Bakterienstämmen, die in unterschiedlichen Minimalmedien mit Isomaltulosemelasse kultiviert wurden, bestimmt. Hierbei wurden 10 ml PSA-Vorkultur über 5 Tage oder 7 Tage in 90 ml M1- oder M2-Medium, jeweils versetzt mit 10% Isomaltulosemelasse, aerob bei 24°C inkubiert und die Gesamtbiomasse nach der Ernte bestimmt (siehe Fig. 3 a) und b), jeweils 1. und 2. Säule). Bei weiteren Ansätzen wurden die Kultivierungsbedingungen nach der 5-tägigen Inkubation von aerob auf anaerob umgestellt und bei unterschiedlichen Temperaturen für 48 h weiter inkubiert (24 °C, 30°C oder 37°C, siehe Fig. 3 a) und b), jeweils 3. bis 5. Säule). Im Anschluss wurde ebenfalls das Gewicht der Gesamtbiomasse bestimmt. Zu weiteren Ansätzen wurden nach der 5-tägigen Inkubation Bakterienvorkulturen (ca. 6*10$^9$ CFU/ml) bestehend aus *P. shermanii* und *P. freudenreichii* oder *L. reuteri* zugegeben, auf anaerobe Bedingungen umgestellt und ein Temperaturshift auf 30 °C bzw. 37 °C vollzogen und für 48 h bei diesen Bedingungen kultiviert (siehe Fig. 3 a) und b), jeweils 6. bis 7. Säule). Anschließend erfolgte wie bei allen Ansätzen die Bestimmung der Gesamtbiomasse. Insgesamt wiesen Kulturen in M2-Medium über einen Zeitraum von 5 Tagen eine höhere Pilzbiomasse auf als Kulturen in M1-Medium. Allerdings konnte die Biomasse innerhalb von 7 Tagen Inkubation im M1-Medium weiter gesteigert werden, während dies für das M2-Medium nicht der Fall war. Sowohl im M1- als auch im M2-Medium konnten somit Gesamtbiomassen von bis zu 49 g / 100 ml, gemessen an der Trockenmasse, durch Kultivierung der Basidiomyceten vor Zugabe der Bakterienarten erzielt werden.

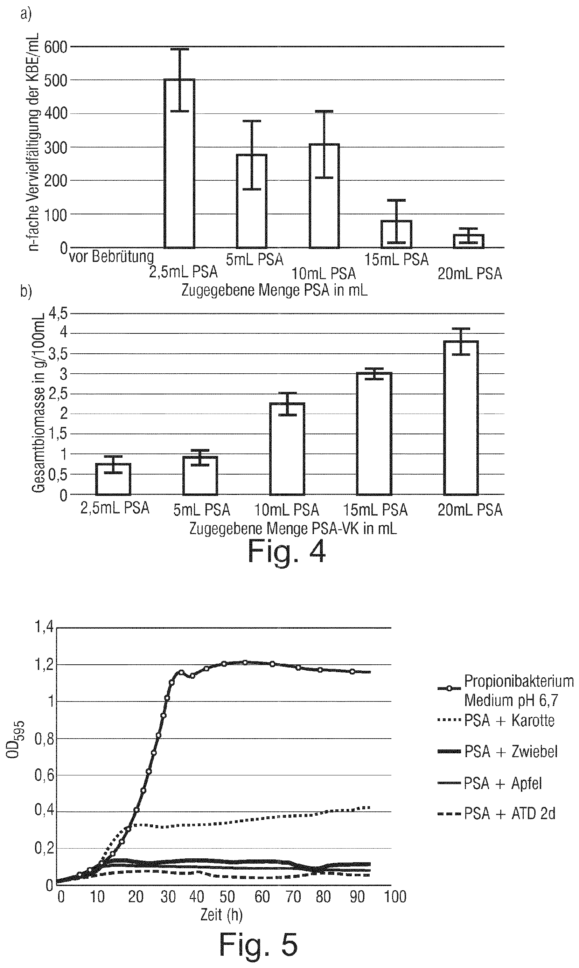

Weiterhin wurde der Einsatz unterschiedlicher Mengen an PSA-Pilzvorkultur bei gleichbleibender Bakterienmenge (1 ml, ca. 6 $^*$ 10$^9$ CFU/ml) getestet. Hierzu wurden 10 ml PSA-Vorkultur über 7 Tage in 90 ml M1-Medium versetzt mit 10% Isomaltulosemelasse aerob inkubiert. Im Anschluss wurde 1 ml einer Vorkultur Propionibakterien (ca. 6*10$^9$) CFU/ml) zugegeben, auf anaerobe Bedingungen umgestellt, ein Temperaturshift auf 30 °C vollzogen sowie für 48 h bei diesen Bedingungen kultiviert (siehe Fig. 4). Hierdurch konnte gezeigt werden, dass steigende Mengen an PSA zu höherer Gesamtbiomasse führen, aber das Bakterienwachstum inhibieren.

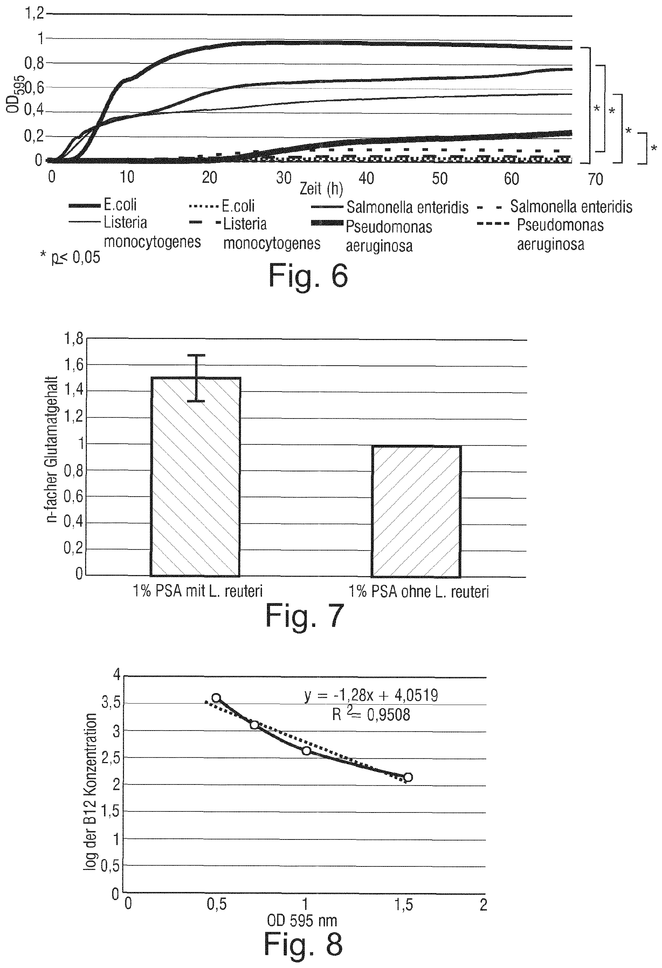

In weiteren Versuchsreihen wurde das Wachstum von Bakterien auf *Pleurotus sapidus* (PSA)-Überständen bestimmt (siehe Fig. 5). Hierzu wurde zunächst PSA auf verschiedenen Agrarnebenströmen kultiviert und geerntet. Die Kultur auf Isomaltulose wurde in M2-Medium über 5 Tage vor der Ernte gehalten, die Kulturen auf Karottentrester, Zwiebeltrester und Apfeltrester wurden in M1-Medium über 4 Tage vor der Ernte gehalten. Bei einem weiteren Ansatz wurde die Kultur in M1-Medium auf Apfeltrester über 2 Tage vor der Ernte gehalten (siehe Fig. 5, "ATD 2d"). Die zur Kultivierung verwendete Medien wurden nach der Ernte des Pilzmycels zur Kultivierung von *P. freudenreichii supspez. shermanii* verwendet. In einem Kontrollversuch wurde *P. freudenreichii supspez. shermanii* auf Optimalmedium (Propionibakterium-Medium) kultiviert. Hierbei zeigte sich, dass *P. freudenreichii supspez. shermanii* kaum auf PSA-Kulturüberständen wuchs. Ebenso zeigten weitere Bakterienarten nur äußerst geringes Wachstum auf PSA-Kulturüberständen basierend auf Apfeltrester als Agrarnebenstrom nach 5 Tagen Kultivierung des PSA-Mycels und anschließender Ernte (siehe Fig. 6, gestrichelte Linien) im Vergleich zum Wachstum auf dem jeweiligen Optimalmedium (siehe Fig. 6, durchgehende Linien).

Diese Ergebnisse zeigen, dass insbesondere die Form der Co-Kultivierung mit Basidiomyceten zu besonders hohen Bakterienteilungsraten führt. Überraschenderweise werden signifikant geringere Bakterienteilungsraten bei der Kultivierung auf Basidiomyceten-Überständen, d.h. auf verbrauchten Medien ohne Basidiomyceten, erreicht. Bereits nach 2 Tagen PSA-Kultivierung auf Apfeltrester inhibiert das verbrauchte Medium das Wachstum von *P. freudenreichii supspez. shermanii* deutlich (siehe Fig. 5), wohingegen in der Co-Kultur auch nach 5 Tagen PSA-Kultivierung vor Zugabe der Bakterien ein deutliches Bakterienwachstum erzielt wird (vgl. Fig. 2-4).

**Beispiel 3: Umwandlung des im *Pleurotus* sapidus-Myzel enthaltenen Glutamins ins Glutamat durch *L. reuteri***

1% gefriergetrocknetes Pilzmyzel wurde mit und ohne *L. reuteri* über Nacht bei 37°C inkubiert und am nächsten Tag nach Aufschluss der Glutamatanteil bestimmt. Die Bestimmung des Glutamatgehaltes in gefriergetrocknetem zuvor mittels Enzymen aufgeschlossenem *Pleurotus sapidus* (PSA)-Myzel, führte zur Detektion einer gesteigerten Umsetzung von Glutamin nach Fermentation durch *L. reuteri*. Nach einer Bestimmung in Triplikaten konnte eine durchschnittliche Steigerung des Glutamatgehaltes um 52% festgestellt werden (siehe Fig. 7).

**Beispiel 4: Bestimmung von Vitamin B12 in Co-Kulturen aus *Pleurotus sapidus* und Vitamin-B12 produzierenden Bakterienstämmen sowie nur in den Bakterienkulturen**

10 ml *Pleurotus sapidus* (PSA)-Vorkultur wurden in 90 ml M1-Medium, versetzt mit 10 % Isomaltulose über 7 Tage schüttelnd bei 24°C inkubiert. Im Anschluss wurde 1 ml *L. reuteri* oder *P. freudenreichii subsp. freudenreichii* und *shermanii* zugegeben (1 ml, ca. $6 * 10^9$ CFU/ml) und für 48 h bei 30°C bzw. 37°C unter anaeroben Bedingungen weiter inkubiert. Die Kulturen wurden nach Inkubation zentrifugiert, das Pellet in $H_2O$ gelöst und zusammen mit Glaskugeln im Verhältnis 1:2 (Glaskugeln/Kultur) sonifiziert für 10 min bei 60% Amplitude (1 min puls, 1 min Pause). Anschließend erfolgte die Umwandlung aller Cobalamine durch Zugabe von 10%-iger Kaliumcyanidlösung mit einer Endkonzentration von 2%. Nach 10 min Inkubation bei Raumtemperatur erfolgte eine erneute Zentrifugation und der Überstand wurde mittels ELISA auf Vorlage von Cyanocobalamin analysiert.

Für die Berechnung der in den Kulturen erhaltenen Mengen an Vitamin B12 wurde eine Standardkurve mit definierten Mengen an Cyanocobalamin angefertigt (siehe Fig. 8), anhand derer die in den Kulturen vorhandenen Mengen an Vitamin B12 ermittelt wurden.

In der folgenden Tabelle sind die vorhandenen Gehalte an Vitamin B12 aus den Co-Kulturen dargestellt. Geht man davon aus, dass im Endprodukt z.B. veganer Bratwurst auf 1 kg 25 g Protein verwendet werden, beträgt der Vitamin B12- Gehalt pro 100 g Bratwurst etwa 0,3 $\mu$g.

**Tab. g:** Vitamin B12- Gehalt in Co-Kulturen aus Basidiomyceten und Bakterien.

| Organismen der Co-Kultur | OD bei 450 nm | Log (Vit. B12-Konz.) | Konz. an Vit. B12 (pg/ml) | $\mu$g Vit. B12 in 100 ml Co-Kultur | Menge der CO-Kultur zur Deckung des Tagesbedarfs an Vit B12 (l) | Menge Trockenmasse zur Deckung des Tagesbedarfs an Vit B12 (g) |
|---|---|---|---|---|---|---|
| *PSA, L. reuteri* | 0,229 | 3,76 | 5738,25 | 0,057 | 5,22 | 29,8 |
| *PSA+ Propiobacterium freudenreichii sups. freudenreichii u. shermanii* | 0,2521 | 3,73 | 5360,58 | 0,107 | 2,79 | 15,98 |

Der Vitamin B12-Gehalt in den Bakterienkulturen ohne Basidiomyceten wurde ebenfalls erfasst und lag deutlich höher (siehe Tab. 4). Hierzu wurden die Bakterienkulturen entweder über 2 Tage anaerob kultiviert und im Anschluss über 24 h aerob oder 3 Tage anaerob bei der jeweiligen Optimaltemperatur des Bakterienstamms kultiviert. Der Bakteriengehalt lag bei ca. $6*10^9$ CFU/ ml. Allerdings würde eine solche Produktion deutlich aufwendiger und kontaminationsanfälliger sein, weshalb der Ansatz der Co-Kultur verfolgt wird.

**Tab.4:** Vitamin B12- Gehalt in unterschiedlich kultivierten Kulturen von Vitamin B12-produzierenden Bakterienstämmen.

| Bakterium | Kultivierung | Konz. Vit. B12 (ng/ml) in Kultur | Kulturmenge, um Tagesbedarf an Vit B12 zu decken | Konz. Vit. B12 im Bakterienpellet ($\mu$g/100g) | Pelletmenge für Deckung des Tagesbedarfs (g) |
|---|---|---|---|---|---|
| *L. reuteri* | 2 d anaerob, 1d aerob | 12,4 | 4,03 | n.a. | n.a |
| *L. reuteri* | 3 d anaerob | 10,09 | 7,42 | 3 | 100 |
| *P. freudenreichii subs. freudenreichii und shermanii* | 2 d anaerob, 1d aerob | 12,69 | 3,93 | n.a. | n.a. |
| *P. freudenreichii subs. freudenreichii und shermanii* | 3 d anaerob | 13,99 | 5,35 | 3,23 | 92,88 |

**Beispiel 5: Quantifizierung des Pilzanteils im Lyophilisat über Ergosterol am Beispiel der Kultivierung von PSA auf Apfeltrester**

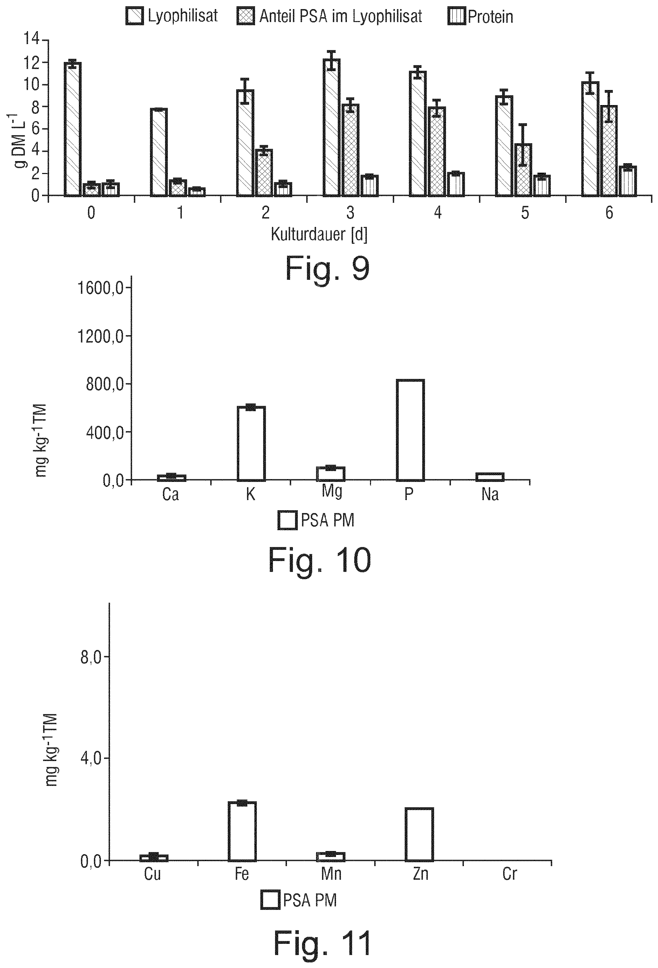

Der Proteingehalt und der Anteil an *Pleurotus sapidus* (PSA) in der Gesamtbiomasse wurde über 6 Tage gemessen. Hierbei wurde der Anteil an PSA über eine Ergosterolmessung bestimmt. Der Anteil an PSA sowie der Proteingehalt stiegen mit der Kulturdauer an. Bei der Gesamtauswaage des Myzels war ab Tag 4 eine leicht abnehmende Tendenz zu erkennen (siehe Fig. 9). Der Anteil an PSA im Lyophilisat betrug zum Erntezeitpunkt ca. 80 %.

**Beispiel 6: Ergebnisse der Bestimmung von Mineralstoffen und Zuckern in PSA-Myzel, kultiviert auf Isomaltulosemelasse sowie Darstellung der Substratzusammensetzung**

Der Umsatz von Glucose und Fructose aus dem Isomaltulosemelasse (Palatinosemelasse/PM)-Substrat durch *Pleurotus sapidus* (PSA) sowie aus Karottentrester (K) durch *Lentinula edodes* (LED) wurde quantifiziert (siehe Tab. 5).

**Tab. 5:** Umsetzung von D-Glucose und D-Fructose aus dem Substrat.

| Basidiomycetenart | Umsatz D-Glucose aus dem Substrat [%] | Umsatz D-Fructose aus dem Substrat [%] |
|---|---|---|
| PSA - PM | 75,2$\pm$ 0,2 | 68,1$\pm$ 0,3 |
| LED - K | 89,6$\pm$ 0,3 | 98,9$\pm$ 0,0 |

Der Gehalt von Glucose und Fructose im Pilzmyzel sowie in dem verwendeten Substrat wurde bestimmt (siehe Tab. 6).

Tab. 6: Bestimmung von D-Glucose und D-Fructose im Pilzmyzel und Substrat.

| Probe | D-Glucose [g(100 g TM)$^{-1}$] | D-Fructose [g (100 g TM)$^{-1}$] |
|---|---|---|
| PSA PM | 4,9$\pm$ 0,0 | 9,4$\pm$ 0,2 |
| Isomaltulosemelasse | 12,4$\pm$ 1,7 | 19,0$\pm$ 2,1 |
| LED K | 0,3$\pm$ 0,0 | 0,1$\pm$ 0,0 |
| Karottentrester | 2,4$\pm$ 0,1 | 7,0$\pm$ 0,0 |

**Tab. 7:** Zusammensetzung an Sacchariden und Schwermetallbelastung in *Pleurotus sapidus* (PSA) kultiviert auf Apfeltrester (ATD), im Restsubstrat ATD sowie Saccharidzusammensetzung in PSA kultiviert auf Isomaltulosemelasse (PM).

| PSA ATD | Konzentration [g/(100g TM)$^{-1}$] | ATD Substrat | Konzentration [g/(100g TM)$^{-1}$] |
|---|---|---|---|
| Chitin | 6,54 $\pm$ 1,79 | | |
| Gesamtglucan | 9,22 $\pm$ 0,16 | Gesamtglucan | 4,39 $\pm$ 0,97 |

(fortgesetzt)

| PSA ATD | Konzentration [g/(100g TM)$^{-1}$] | ATD Substrat | Konzentration [g/(100g TM)$^{-1}$] |
|---|---|---|---|
| Beta-Glucan | 5,59 | Beta-Glucan | 3,42 |
| Alpha-Glucan | 3,64 $\pm$ 0,09 | Alpha-Glucan | 0,97 $\pm$ 0,05 |
| Glucose | 0,55 $\pm$ 0,02 | Glucose | 1,5 $\pm$ 0 |
| Fructose | 0,07 $\pm$ 0,01 | Fructose | 5,15 $\pm$ 0,02 |
| Saccharose | 0,34 $\pm$ 0,01 | Saccharose | 0,32 $\pm$ 0,06 |

| PSA ATD | Konzentration [$\mu$g/(kg TM$^{-1}$)] | ATD Substrat | Konzentration [$\mu$g/(kg TM$^{-1}$)] |
|---|---|---|---|
| Blei | n.n. | Blei | n.n. |
| Cadmium | 13,3 $\pm$ 1,9 | Cadmium | 12,3 $\pm$ 1,9 |
| Quecksilber | n.n. | Quecksilber | n.n. |

| PSA PM | Konzentration [g/(100g TM)$^{-1}$] | | |
|---|---|---|---|
| Alpha-Glucan | 6,1 | | |
| Beta-Glucan | 18,7 | | |
| Gesamtglucan | 24,8 | | |

## Beispiel 7: Aminosäureprofil

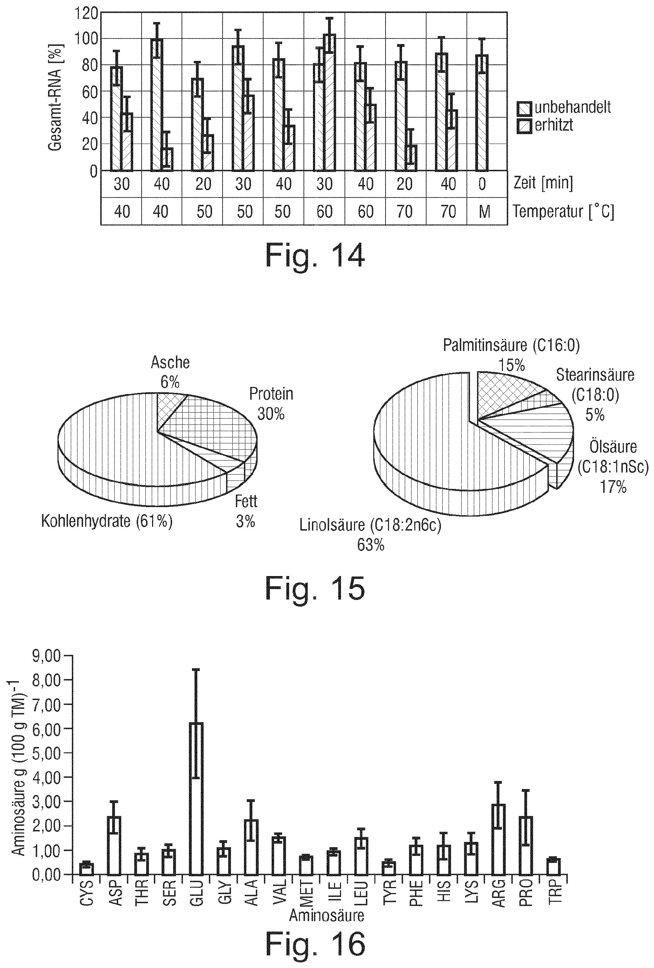

Der Gesamtaminosäuregehalt von 29,41 g (100 g TM)$^{-1}$ wurde als Summe der einzelnen Aminosäuren berechnet. Vergleicht man diesen Gehalt mit dem Gesamtrohproteingehalt nach Kjeldahl (27,41 g (100 g TM)$^{-1}$), so korrelieren die beiden Werte sehr gut miteinander. In Summe ergibt sich ein Aminosäurespektrum von insgesamt 18 Aminosäuren, darunter die 8 essentiellen sowie die beiden semiessentiellen Aminosäuren Arginin und Histidin (siehe Fig. 16).

## Beispiel 8: Vitamin D2-Produktion

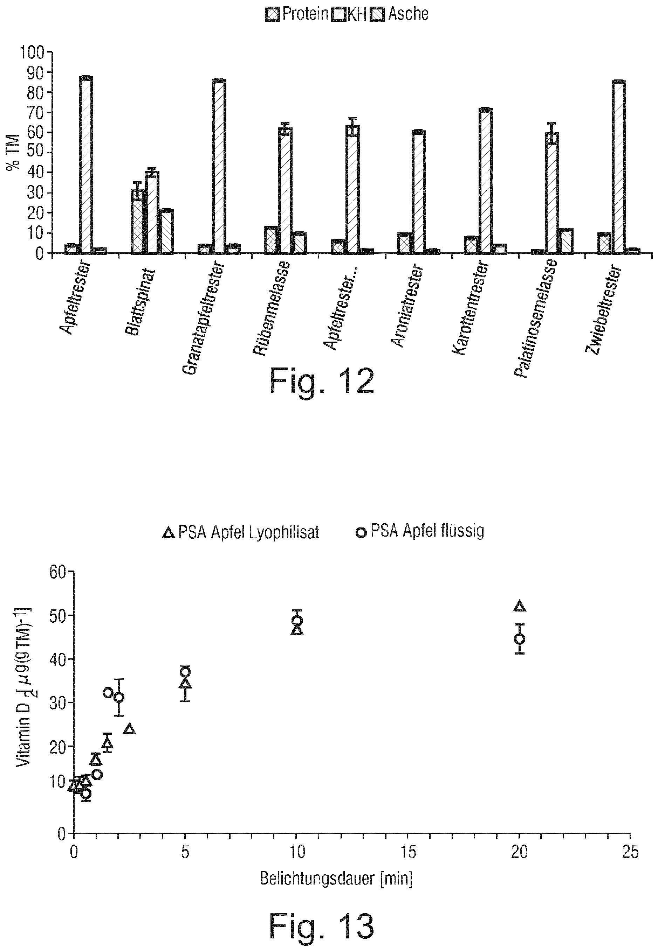

In zwei Versuchsreihen wurde getestet, ob es Unterschiede in der Belichtung des bereits lyophilisierten Pilzmyzels im Vergleich zur Belichtung der gesamten Submerskultur in flüssigem Zustand gibt. Der Durchmesser der Kristallisierschalen, in denen belichtet wurde, betrug 9,5 cm beim Lyophilisat (Probenhöhe: 0,8 cm) und 19,8 cm bei Belichtung der flüssigen Kultur (Probenhöhe: 1 cm). Bereits eine Belichtung von wenigen Sekunden reichte aus, um so viel Vitamin D2 zu produzieren, dass der Tagesbedarf von 5 $\mu$g bei einem Verzehr von 1 g trockenem Pilzmyzel bereits überschritten wird (Fig. 13). Für die spätere Produktion kann daher unbelichtetes mit belichtetem Myzel vermischt werden, um den Tagesbedarf an Vitamin D2 nicht zu überschreiten. Die Bestrahlung von Lyophilisat, das der Kultivierung von PSA auf Isomaltulose entstammt und 10 min mit UV-Licht bei einem Lampenabstand von 10 cm erfolgte, ergab einen Gehalt an Vitamin D2 von 0,2 $\mu$g/g Trockenmasse.

## Beispiel 9: Reduktion des RNA-Gehalts

Durch Hitzebehandlung einer PSA-Kultur auf Isomaltulosemelasse am Ende der Inkubationszeit konnte der RNA-Gehalt im Vergleich zu den unbehandelten Proben deutlich gesenkt werden. Insbesondere die Kombinationen aus 40 Minuten bei 40 °C und 20 Minuten bei 70 °C führten zur deutlichen Reduktion der Gesamt-RNA (18 S- und 28 S-RNA). Damit kann gesundheitlicher Mehrwert des Produktes erzielt werden, was insbesondere für Risikogruppen unter den Verbrauchern relevant ist (siehe Fig. 14).

## Beispiel 10: Technofunktionelle Untersuchungen

Die folgenden Abkürzungen werden im Folgenden verwendet: "g" = getrockneter Trester (ansonsten Feuchttrester); "[Zahl] T" = Anzahl der Tage der Kultivierung.

Weiterhin werden die folgenden Abkürzungen für die Basidiomycetenstämme, die Nebenströme sowie die Medien verwendet:

**Tab. 8:** Verwendete Stämme und Nebenströme.

| Stamm | Nebenstrom |
|---|---|
| AAE (*Agrocybe aegerita*) | KTD (Karottentrester Döhler) |
| LED (*Lentinula edodes*) | KT (Karottentrester) |
| LSU (*Laetiporus sulphureus*) | AT (Apfeltrester) |
| PSA (*Pleurotus sapidus*) | ZT (Zwiebeltrester |
| PEO (*Pleurotus roseus*) | GA (Granatapfeltrester) |
| SRU (*Stropharia rugosoannulata*) | Aro (Aroniatrester) |
| WCO (*Wolfiporia cocos*) | BS (Blattspinat) |
| | PM (Isomaltulosemelasse) |

**Tab. 9:** Zur Medienoptimierung verwendete Flüssigmedien. SE: Spurenelementlösung, Na-Aspartat: L-Asparaginsäure Mononatriumsalz Monohydrat.

| | Na-Aspartat [g L$^{-1}$] | NH$_4$NO$_3$ [g L$^{-1}$] | (NH$_4$)$_2$SO$_4$ [g L$^{-1}$] | KH$_2$PO$_4$ [g L$^{-1}$] | MgSO$_4$ [g L$^{-1}$] | SE-Lsg. [mL L$^{-1}$] |
|---|---|---|---|---|---|---|
| M1 | 6,2 | 2,4 | - | 1,5 | 0,5 | 1,0 |
| JA 1 | 3,1 | 2,4 | - | 1,5 | 0,5 | 1,0 |
| JA 2.1 | 6,2 | - | 4,0 | 1,5 | 0,5 | 1,0 |
| JA 2.2 | 6,2 | - | 2,4 | 1,5 | 0,5 | 1,0 |
| JA 3 | 6,2 | 2,4 | - | 0,5 | 0,5 | 1,0 |
| JA 4 | 3,1 | - | 2,4 | 0,5 | 0,5 | 1,0 |
| VE-H$_2$O mit SE | - | - | - | - | - | 1,0 |
| | - | - | - | - | - | - |

**Tab. 10:** Zur Medienoptimierung verwendete Flüssigmedien. SE: Spurenelementlösung.

| | Hefeextrakt [g L$^{-1}$] | Pepton (Soja) [g L$^{-1}$] | NH$_4$NO$_3$ [g L$^{-1}$] | (NH$_4$)$_2$SO$_4$ [g L$^{-1}$] | KH$_2$PO$_4$ [g L$^{-1}$] | MgSO$_4$ [g L$^{-1}$] | SE-Lsg. [mL L$^{-1}$] |
|---|---|---|---|---|---|---|---|
| M2 | 3,0 | - | - | - | 1,5 | 0,5 | 1,0 |
| M2a | 3,0 | - | 2,4 | - | 1,5 | 0,5 | 1,0 |
| M2b | 3,0 | - | - | - | 0,5 | 0,5 | 1,0 |
| M3 | - | 3,0 | - | - | 1,5 | 0,5 | 1,0 |
| M3a | 3,0 | 3,0 | - | - | 1,5 | 0,5 | 1,0 |
| M3b | - | 3,0 | - | - | 0,5 | 0,5 | 1,0 |

Wasserbindekapazität:

Die Proben LSU_KTD_16T_M1_20.8.15, LSU_KTD_16T_M2_20.8.15, PSA_AT_M1_G_5.8.15, PSA_KT_G_5.8.15, PSA_KT_G_5.8.15_II, PSA_KTD_16T_M2_20.8.15 und PSA_ZT_20.2.15 weisen alle eine hohe Wasserbindekapazität auf. Der Einfluss des Agrarnebenstromes ist jedoch hierbei nicht zu unterschätzen, da besonders Fasern aus Karotten, Äpfeln oder Zwiebeln hohe kapillare Kräfte haben, die auch eine Wasserbindekapazität besitzen. Betrachtet man den Basidiomyceten *Pleurotus sapidus* auf Isomaltulosemelasse (PSA_PM), so kann man von der verwendeten Isomaltulosemelasse keine Funktionalität erwarten. Die Eigenschaften des Myzels aus PSA_PM können mit Erbsenproteinisolat verglichen werden.

**Tab. 11:** Wasserbindekapazitäten.

| Name | WBC/Protein*100 | WBC [mL/g] | Protein [%] |
|---|---|---|---|
| 112541_Erbsenproteinisolat | 3,50 | 3,2 | 90,1 |
| 113764_Sojakonzentrat | 5,87 | 4,1 | 69,8 |
| 118770_Sojaisolat | 7,45 | 6,7 | 90,2 |

(fortgesetzt)

| Name | WBC/Protein*100 | WBC [mL/g] | Protein [%] |
|---|---|---|---|
| AAE_AT_Gesamt6 | 30,45 | 5,1 | 16,7 |
| AAE_ATD_G_10.8.15 | 31,10 | 5,8 | 18,6 |
| AAE_ATD_G_10.8.15_II | 35,73 | 6,6 | 18,6 |
| AAE_BS_Gesamt2 | 12,37 | 3,2 | 26,1 |
| AAE_GA_Gesamt7 | 27,56 | 5,1 | 18,6 |
| AAE_KTD_16T_M1_20.8.15 | 23,04 | 5,0 | 21,8 |
| AAE_KTD_16T_M2_20.8.15 | 28,42 | 5,5 | 19,2 |
| AAE_P100_M1_16.2.15 | 8,43 | 1,1 | 13,3 |
| LED_ATD_G_10.8.15 | 18,58 | 3,8 | 20,4 |
| LED_ATD_G_10.8.15_II | 22,20 | 4,5 | 20,4 |
| LED_GA_16.4.15 | 19,94 | 3,2 | 16,3 |
| LED_GA_16.4.15_II | 21,50 | 3,5 | 16,3 |
| LED_KTD_6T_M1_20.8.15 | 12,63 | 3,4 | 27,2 |
| LED_KTD_M1_8.15 | 14,99 | 4,0 | 27,0 |
| LED_P100_16T_M2_30.3.15 | 16,39 | 3,9 | 24,0 |
| LSU_AT_Gesamt4 | 34,31 | 5,3 | 15,5 |
| LSU_KTD_16T_M1_20.8.15 | 76,15 | 7,0 | 9,1 |
| LSU_KTD_16T_M2_20.8.15 | 83,58 | 7,3 | 8,8 |
| PSA_Aro_G_18.8.15 | 35,67 | 4,2 | 11,6 |
| PSA_Aro_G_18.8.15_II | 30,59 | 3,6 | 11,6 |
| PSA_AT_Gesamt1 | 22,670 | 4,5 | 20,0 |
| PSA_AT_M1_G_5.8.15 | 34,37 | 7,1 | 20,7 |
| PSA_BS1_Gesamt9 | 21,10 | 5,9 | 28,0 |
| PSA_BS2_31.3.15 | 13,97 | 3,9 | 28,0 |
| PSA_GA_Gesamt3 | 29,79 | 3,3 | 11,1 |
| PSA_KT_G_5.8.15 | 28,91 | 7,3 | 25,1 |
| PSA_KT_G_5.8.15_II | 29,28 | 7,3 | 25,1 |
| PSA_KTD_16T_M2_20.8.15 | 23,29 | 7,5 | 32,1 |
| PSA_KTD_3T_M2b_29.9.15 | 13,20 | 3,1 | 23,5 |
| PSA_KTD_4T_M2_14.9.15 | 7,24 | 2,2 | 30,7 |
| PSA_KTD_6T_M2a_14.9.15 | 9,79 | 2,1 | 21,9 |
| PSA_KTD_6T_M3_14.9.15 | 10,82 | 2,2 | 20,2 |
| PSA_KTD_6T_M3a_21.9.15 | 10,51 | 1,9 | 18,5 |
| PSA_KTD_9T_M3b_21.9.15 | 19,29 | 2,4 | 12,4 |
| PSA_P100_M1M2_21.8.15 | 12,64 | 2,5 | 20,1 |
| PSA_PM_M2 | 11,560 | 2,7 | 23,8 |
| PSA_ZT_20.2.15 | 71,70 | 7,8 | 10,9 |
| PSA_ZT_24.11.14 | 17,31 | 4,9 | 28,4 |
| SRU_BS_Gesamt5 | 14,13 | 4,8 | 34,0 |

(fortgesetzt)

| Name | WBC/Protein*100 | WBC [mL/g] | Protein [%] |
|---|---|---|---|
| SRU_GA_Gesamt8 | 59,40 | 4,2 | 7,0 |
| WCO_Aro_G_18.8.15 | 27,15 | 3,5 | 12,9 |
| WCO_Aro_G_18.8.15_II | 26,31 | 3,4 | 12,9 |
| WCO_KTD_24T_M1_20.8.15 | 40,77 | 5,4 | 13,2 |
| WCO_ZT_24.11.14 | 43,81 | 6,1 | 14,0 |
| WCO_ZT_25.11.14_II | 25,66 | 3,6 | 14,0 |

Ölbindekapazität:

Einige Agrarnebenströme binden durch ihre kapillaren Kräfte (hervorgerufen durch den Faseranteil) mehr Öl als andere. Vergleicht man die Wasserbindekapazität von *Pleurotus sapidus* auf Isomaltulosemelasse (PSA_PM) mit der von pflanzlichen Proteinen, so hat das Basidiomyceten-Myzel eine deutlich höhere Ölbindekapazität. Diese Funktionalität ist eventuell durch die Bildung von $\beta$-Glucanen und Chitin durch den Basidiomyceten zu erklären.

**Tab. 12:** Ölbindekapazitäten.

| | ÖBC/Protein*100 | ÖBC [ml/g] | Protein [%] |
|---|---|---|---|
| 112541_Erbsenproteinisolat | 1,09 | 0,9 | 90,1 |
| 113764_Sojakonzentrat | 2,06 | 1,1 | 69,8 |
| 118770_Sojaisolat | 1,14 | 0,9 | 90,2 |
| AAE_AT_Gesamt6 | 35,43 | 5,1 | 16,7 |
| AAE_ATD_G_10.8.15 | 41,30 | 6,9 | 18,6 |
| AAE_ATD_G_10.8.15_II | 46,49 | 7,8 | 18,6 |
| AAE_BS_Gesamt2 | 14,56 | 3,1 | 26,1 |
| AAE_GA_Gesamt7 | 38,15 | 6,3 | 18,6 |
| AAE_KTD_16T_M1_20.8.15 | 32,18 | 6,2 | 21,8 |
| AAE_KTD_16T_M2_20.8.15 | 35,22 | 6,0 | 19,2 |
| AAE_P100_M1_16.2.15 | 24,95 | 2,5 | 13,3 |
| LED_ATD_G_10.8.15 | 40,11 | 7,4 | 20,4 |
| LED_ATD_G_10.8.15_II | 50,49 | 9,5 | 20,4 |
| LED_GA_16.4.15 | 29,08 | 3,9 | 16,3 |
| LED_GA_16,4.15_II | 28,98 | 3,9 | 16,3 |
| LED_KTD_6T_M1_20.8.15 | 20,75 | 4,9 | 27,2 |
| LED_KTD_M1_8.15 | 25,83 | 6,2 | 27,0 |
| LED_P100_16T_M2_30.3.15 | 12,94 | 2,4 | 24,0 |
| LSU_AT_Gesamt4 | 40,84 | 5,5 | 15,5 |
| LSU_KTD_16T_M1_20.8.15 | 90,30 | 7,3 | 9,1 |
| LSU_KTD_16T_M2_20.8.15 | 102,44 | 8,1 | 8,8 |
| PSA_Aro_G_18.8.15 | 37,46 | 3,5 | 11,6 |
| PSA_Aro_G_18.8.15_II | 35,75 | 3,3 | 11,6 |
| PSA_AT_Gesamt1 | 23,18 | 3,8 | 10,0 |
| PSA_AT_M1_G_5.8.15 | 37,46 | 7,0 | 20,7 |

(fortgesetzt)

| | ÖBC/Protein*100 | ÖBC [ml/g] | Protein [%] |
|---|---|---|---|
| PSA_BS1_Gesamt9 | 22,61 | 5,6 | 28,0 |
| PSA_BS2_31.3.15 | 13,57 | 3,1 | 28,0 |
| PSA_GA_Gesamt3 | 36,00 | 3,1 | 11,1 |
| PSA_KT_G_5.8.15 | 32,70 | 7,5 | 25,1 |
| PSA_KT_G_5.8.15_II | 34,17 | 7,8 | 25,1 |
| PSA_KTD_16T_M2_20.8.15 | 18,91 | 5,4 | 32,1 |
| PSA_KTD_3T_M2b_29.9.15 | 27,97 | 5,8 | 23,5 |
| PSA_KTD_4T_M2_14.9.15 | 14,97 | 3,9 | 30,7 |
| PSA_KTD_6T_M2a_14.9.15 | 20,94 | 3,8 | 21,9 |
| PSA_KTD_6T_M3_14.9.15 | 20,36 | 3,3 | 20,2 |
| PSA_KTD_6T_M3a_21.9.15 | 23,90 | 3,6 | 18,5 |
| PSA_KTD_9T_M3b_21.9.15 | 33,44 | 3,3 | 12,4 |
| PSA_P100_M1M2_21.8.15 | 20,17 | 3,3 | 20,1 |
| PSA_PM_M2 | 19,03 | 3,8 | 23,8 |
| PSA_ZT_20.2.15 | 54,84 | 5,1 | 10,9 |
| PSA_ZT_24.11.14 | 21,38 | 5,4 | 28,4 |
| SRU_BS_Gesamt5 | 17,95 | 5,4 | 34,0 |
| SRU_GA_Gesamt8 | 86,80 | 5,2 | 7,0 |
| WCO_Aro_G_18.8.15 | 32,93 | 3,4 | 12,9 |
| WCO_Aro_G_18.8.15_II | 30,77 | 3,1 | 12,9 |
| WCO_KTD_24T_M1_20.8.15 | 49,59 | 5,7 | 13,2 |
| WCO_ZT_24.11.14 | 32,42 | 3,7 | 14,0 |

Bestimmung der Biologischen Wertigkeit:

Die Bestimmung der biologischen Wertigkeit ergibt für *Pleurotus sapidus,* auf Isomaltulosemelasse kultiviert, ein Ergebnis von 73. Im Vergleich zu anderen Lebensmitteln ist dieser Wert überraschend hoch (siehe Tab. 13).

**Tab. 13:** Vergleich der biologischen Wertigkeit unterschiedlicher Lebensmittel

| Protein | biologische Wertigkeit |
|---|---|
| Hühnerei | 100 |
| Schweinefleisch | 85 |
| Rindfleisch | 80 |
| Geflügel | 80 |
| Kuhmilch | 72 |
| Sojaprotein | 81 |
| Roggenmehl (82% Ausmahlung) | 78 |
| Kartoffel | 76 |
| Bohnen | 72 |
| Reis | 66 |

(fortgesetzt)

| Protein | biologische Wertigkeit |
|---|---|
| Weizeriniehl (62% Ausmahlung) | 47 |

**Beispiel 11: Herstellung und Rezeptur einer veganen Bratwurst**

Bräunung und Bräunungsgeschmack werden in Verbindung mit reduzierenden Zuckern und Myzel von Basidiomyceten, gewonnen aus Submerskultur, mit verschiedenen kohlenhydratreichen Agrarnebenströmen gebildet. Hierzu wurden 13 verschiedene Bratwürste als fein (I bis IV) oder grobe (VII bis XIII) bzw. einer feinen handelsüblichen "fleischigen" Bratwurst (VII) Variante mit folgenden Benennungen hergestellt:

I: ohne Protein
II: Erbsenproteinisolat
III: Sonnenblumenproteinkonzentrat
IV: Sojaproteinisolat
V: Sojaproteinkonzentrat
VI: *Pleurotus sapidus* auf Isomaltulosemelasse kultiviert (PSA PM)
VII: normale Bratwurst (Fleischprotein)
VIII: ohne Protein
IX: Erbsenproteinisolat
X: Sonnenblumenproteinkonzentrat
XI: Sojaproteinisolat
XII: Sojaproteinkonzentrat
XIII: *Pleurotus sapidus* auf Isomaltulose (PSA PM)

Die feine Variation der veganen Bratwurst (I bis VI) wurde hergestellt aus 2 Emulsionen:

a) Emulsion 1:500 g Emulsion 1 bestehend aus 0,85 g Methylcellulose und 0,15 g Maismehl mit 13,3 g Rapsöl und 347 g Eiswasser. Die Emulsion wurde im Thermomix (Baujahr 2010; TM31) auf Stufe 4 während 5 min hergestellt.
b) Emulsion 2:500g Emulsion 2 bestehend aus 370 g Eiswasser, 85 g Rapsöl, 4g Speisesalz, 1 g Kaliumchlorid, 5 g Citrusfaser, 20 g kappa-Carrageen und 15 g Erbsenstärke instant. Die Emulsion wurde im Thermomix (siehe zuvor) auf Stufe 6 während 5 min hergestellt.

Anschließend wurden beide Emulsionen zusammengefügt und im Thermomix auf Stufe 5 während 2 min emulgiert.
Zu 1.000g Emulsion bestehend aus 500g Emulsion 1 und 500g Emulsion 2 wurden vor dem Schritt der Emulsionsbildung folgende Zutaten zugegeben:

5g/ kg Kochsalz
25 g/kg diverse Proteine bzw. kein Zusatz:
I: ohne Protein
II: Erbsenproteinisolat
III: Sonnenblumenproteinkonzentrat
IV: Sojaproteinisolat
V: Sojaproteinkonzentrat
VI: *Pleurotus sapidus* auf Isomaltulose (PSA PM) und 30g/kg Gewürz mit folgenden Rezeptur:

| | |
|---|---|
| Dextrose fein | 2,100 g |
| Stecte-Speisesalz | 8,000 g |
| Pfeffer weiß gemahlen | 2,500 g |
| Muskatblüte gemahlen | 0,500 g |
| Ingwer gemahlen | 0,800 g |
| Macis Ansatz | 0.400 g |
| Citronensäure E330 | 0.700 g |
| VM SMAK® GOURMET WL | 6,000 g |
| Wurst Aroma vegan | 4.000 g |

(fortgesetzt)

| Gekörnte Brühe oG | 4,900 g |
|---|---|
| Zitronenaroma (Pulver) | 0.100 g |

Die Emulsion und alle genannten Zutaten wurden anschließend im Thermomix (wie zuvor) für weitere 2 min bei Stufe 6 gemischt.

Die handelsübliche Bratwurst (VII normale Bratwurst) wird nach folgender Rezeptur hergestellt:

200 g Schweinefleisch kategorisiert nach GEHA SII
250 g Schweine-Beinfleisch
100 g Schweinebacken
250 g Schweinespeck
200g Eis

Zu 1.000g Fleischbrät bestehend aus den obigen Rohstoffen werden folgende Gewürze und Zusatzstoffe (mit einer Gesamtmenge von 16 g/kg) zugefügt:

| Dextrose fein | 1,254 g |
|---|---|
| Siede-Speisesalz | 1,95 g |
| Phosphat E 450 | 1,05 g |
| Emulgator E471 | 0,675 g |
| Emulgator E472b | 0,075 g |
| Natriumcarbonat E500 | 0,13 g |
| Glucosesirup | 1,15-g |
| Citrat E331 | 1,75 g |
| Pfeffer schwarz Ansatz | 2,8 g |
| Kieselsäure | 0,008 g |
| Macis Oleoresin flü | 0,064 g |
| Ingwer Oleoresin flü | 0,024 g |
| Muskat Ansatz | 0,16 g |
| Kardamom gemahlen | 0,28 g |
| Ingwer entölt gemahlen | 2,96 g |
| Würze | 1,2 g |

Die Bratwurst wird im Kutter der Firma Seydelmann (Baureihe K60) wie folgt hergestellt:
Das Fleisch wird mit Hilfe eines Fleischwolfes der Firma MADO (MEW613) auf die Größe von 3 mm zerkleinert und für 10 Runden mit allen Zutaten bei 3600 Umdrehungen pro Minute gekuttert. Anschließend wird 1/3 des Eises hinzugefügt und für weitere 30 Runden bei 3600 Umdrehungen pro Minute zerkleinert. Der Deckel des Kutters wird von inne gesäubert, das restliche Eis hinzugefügt und bei 3600 Umdrehungen pro Minute bis zu einer Endtemperatur von 10°C fertig gekuttert.

Die grobe Variation der veganen Bratwurst (VIII bis XIII) wurde hergestellt aus 2 Emulsionen und Weizentexturat mit einem anteiligen Wassergehalt:

a) Emulsion 1:500 g Emulsion 1 bestehend aus 0,85 g Methylcellulose und 0,15 g Maismehl mit 133 g Rapsöl und 347 g Eiswasser. Die Emulsion wird im Thermomix auf Stufe 4 während 5 min hergestellt.
b) Emulsion 2:500g Emulsion 2 bestehend aus 370 g Eiswasser, 85 g Rapsöl, 4 g Speisesalz, 1 g Kaliumchlorid, 5 g Citrusfaser, 20 g kappa-Carrageen und 15 g Erbsenstärke instant. Die Emulsion wird im Thermomix bei Stufe 6 während 5 min hergestellt.

Danach werden die beiden Emulsionen zusammengefügt.

900 g Emulsion bestehend aus 450 g Emulsion 1 und 450 g Emulsion 2 und 100g eingeweichtes Weizentexturat (33,35 g Weizentexturat trocken und 66,65 g destilliertes Wasser) wurden im Thermomix bei Stufe 5 während 2 min emulgiert.

Folgende Zutaten wurden vor dem Schritt der Emulsionsbildung hinzugegeben.
5g/ kg Kochsalz
25 g/ kg diverse Proteine bzw. kein Zusatz:

VIII: ohne Protein
IX: Erbsenproteinisolat
X: Sonnenblumenproteinkonzentrat
XI: Sojaproteinisolat
XII: Sojaproteinkonzentrat
XIII: PSA PM

Und 30 g/kg Gewürz mit folgender Rezeptur:

| | |
|---|---|
| Dextrose fein | 2,1 g |
| Siede-Speisesalz | 8,0 g |
| Pfeffer weiß gemahlen | 2,5 g |
| Muskatblüte gemahlen | 0,5 g |
| Ingwer gemahlen | 0,8 g |
| Macis Ansatz | 0,4 g |
| Citronensäure E330 | 0,7 g |
| VM SMAK®Gourmet WL | 6,0 g |
| Wurstaroma vegan | 4,0 g |
| Gekörnte Brühe oG | 4,9 g |
| Zitronenaroma | 0,1 g |

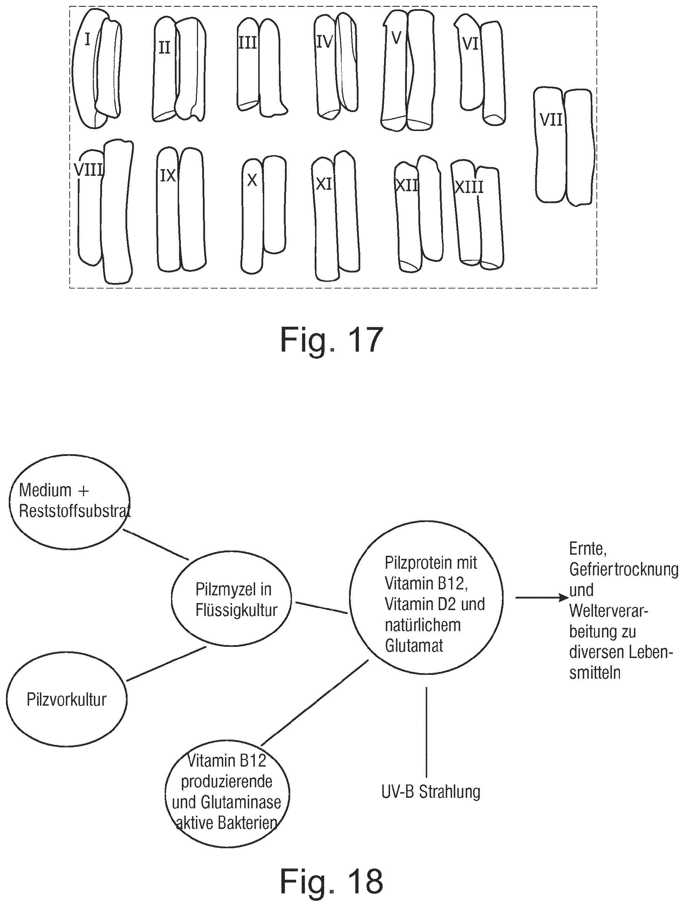

Die diversen Bratwürste (Fig. 17) werden nach thermischer Behandlung während einer Stunde bei 85°C über 12 h bei 2 °C abgekühlt. Nach weiteren 24 h wurden die Bratwürste 3 h bei Raumtemperatur temperiert und im Anschluss bei 175 °C in der Fritteuse während 3 min thermisch behandelt.
Im Vergleich zu pflanzlichen Proteinen hat das proteinreiche Basidiomyceten-Myzel (PSA auf Isomaltulosemelasse) bei der Bräunung in Bratwurst die besten Ergebnisse erzielt. Eine deutlich braunere Farbe im Anschluss an das Frittieren erhöht die Verbraucherakzeptanz, da die meisten anderen pflanzlichen Proteine diese Bräunung nicht zeigen.

**Tab. 14:** Visuelle Einteilung der diversen Bratwürste mit verschiedenen Proteinen zu Fig. 14.

Einteilung der Bräunung

|  | ohne Zusatz von Protein | Erbsenproteinisolat | Sonnenproteinkonzentrat | Sojaproteinisoiat | Sojapro protein-teinkon konzentral | Basidiomyceten myzel | Bratwurst/ Fleisch |
|---|---|---|---|---|---|---|---|
| ohne Weizentexturat | (-) | (+) | (+) | (o) | (o) | (++) | |
| mit Weizentexturat | (-) | (+++) | (++) | (o) | (o) | (+++) | (++) |

(-) = schlechtes Bratverhalten (+) = mäßig gutes Bratverhalten (++) = optimales Bratverhalten nach Verbrauchererwartung (+++) = überaus gutes Bratverhalten, die Zeit kann verkürzt werden (o) = durchschnittliches Bratverhalten

**Tab. 15:** Lxaxb-Messung der Bratwurst nach 3 min bei 175 °C in der Fritteuse.

| | L | [SD] | a | [SD] | b | [SD] |
|---|---|---|---|---|---|---|
| *Ohne Weizentexturat:* | | | | | | |
| ohne Protein | 88,31 | ± 0,11 | 0,35 ± | 0,03 | 2,78 | ± 0,08 |
| Erbsenproteinisolat | 87,55 | ± 0,12 | 0,30 | ± 0,05 | 3,04 | ± 0,09 |
| Sonnenblumenproteinkonz. | 87,41 | ± 0,21 | 0,23 | ± 0,10 | 3,06 | ± 0,10 |
| Sojaproteinisolat | 87,50 | ±0,14 | 0,14 | ±0,02 | 2,80 | ± 0,07 |
| Sojaproteinkonz. | 87,54 | ± 0,06 | 0,18 | ± 0,05 | 3,18 | ± 0,10 |
| PSA_PM_15.07.2015 | 85,76 | ± 0,11 | 0,45 | ± 0,04 | 1,55 | ± 0,12 |
| normale Bratwurst *Mit Weizentexturat:* | 87,31 | ± 0,02 | 0,20 | ± 0,05 | 2,99 | ± 0,03 |
| ohne Protein | 86,35 | ± 0,11 | 0,15 ± | 0,05 | 1,79 | ± 0,10 |
| Erbsenproteinisolat | 85,76 | ± 0,14 | 0,45 | ± 0,04 | 1,55 | ± 0,14 |
| Sonnenblumenproteinkonz. | 86,03 | ± 0,17 | 0,42 | ± 0,08 | 2,14 | ± 0,31 |
| Sojaproteinisolat | 86,42 | ± 0,10 | 0,27 | ± 0,04 | 2,12 | ± 0,24 |
| Sojaproteinkonz. | 86,49 | ± 0,24 | 0,22 | ± 0,06 | 2,08 | ± 0,26 |
| PSA_PM_15.07.2015 | 85,73 | ± 0,12 | 0,35 | ± 0,04 | 1,57 | ± 0,07 |

**Beispiel 12: Co-Kultivierung von *Pleurotus sapidus, P. freudenreichii* und *L. reuteri***

Zu einer *Pleurotus sapidus* (PSA)-Vorkultur wurden Vitamin-B12 produzierende *L. reuteri* oder *P. freudenreichii* Bakterien zugegeben und für 24 h bzw. 48 h aerob bzw. anaerob weiter inkubiert, wie unter Beispiel 2 beschrieben ist. In zwei der Kulturen wurde zusätzlich Glucose zugefügt, bzw. wurde in einer der Kulturen 5,6-Dimethylbenzimidazol (DMB), Bestandteil des Vitamin B12-Komplexes, zugefügt. Die Kulturen wurden nach Inkubation geerntet und die Steigerung der Biomasse bzw. des Vitamin B12-Gehalts, wie oben geschrieben, bestimmt. Insbesondere durch Zugabe von DMB konnte die Gesamtbiomasse sowie der Vitamin B12-Gehalt deutlich gesteigert werden.

**Beispiel 13: Co-Kultivierung unter Verwendung eines vegetarischen Restsubstrates**

Eine Pilz-/Bakterien-Co-Kultivierung gemäß der vorliegenden Erfindung wurde wie oben in Beispiel 2 beschrieben durchgeführt und die Steigerung der Gesamtbiomasse sowie des Bakteriengehalts (in KBE/ml) bestimmt. Dabei wurde einerseits Minimalmedium versetzt mit Palatinose, wie oben beschrieben, und andererseits Minimalmedium versetzt mit dem Lebensmittelnebenstrom Molke verwendet.
Sowohl die Biomasse als auch der Bakteriengehalt konnten durch die Verwendung von Molke als Restsubstrat im Vergleich zu Kultivierungen auf Palatinose gesteigert werden.

**Beispiel 14: Reaktoren zur Co-Kultivierung**

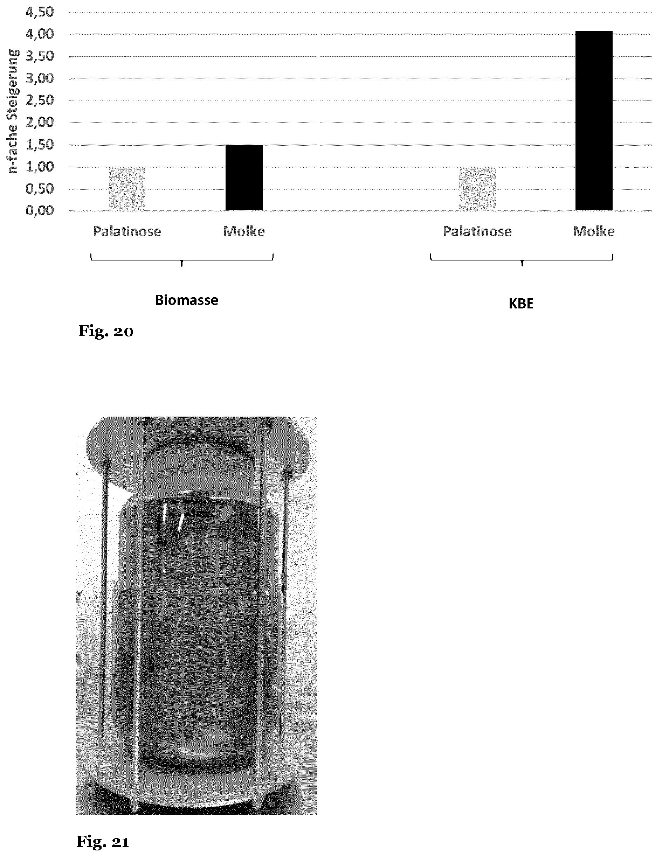

Eine *Pleurotus sapidus* (PSA)-Reinkultur wurde in 4L-Versuchsreaktoren kultiviert. Zwei beispielhafte Ausführungsformen der Versuchsreaktoren sind in Figuren 20 und 21 dargestellt. Durch die Kultivierung in den Versuchsreaktoren, beispielsweise während 62 h, wie in Fig. 20 gezeigt, konnten hohe PSA-Biomassen erzielt werden.

**Patentansprüche**

1.    Verfahren zur Herstellung eines vitamin- und proteinreichen Produkts, umfassend die Schritte:

a) Kultivieren zumindest einer Art aus der Abteilung der Basidiomyceten submers in einem Nährmedium enthaltend zumindest einen kohlenhydrathaltigen Agrarnebenstrom oder Lebensmittelnebenstrom, um ein erstes Kultivierungsprodukt zu erhalten, wobei das erste Kultivierungsprodukt Biomasse der zumindest einen Art aus der Abteilung der Basidiomyceten umfasst;
b) Zugabe zumindest einer Vitamin B12-produzierenden Art der Gattung Propionibacterium und/oder der Gattung Lactobacillus zu dem ersten Kultivierungsprodukt; und
c) Kultivieren der zumindest einen Art der Gattung Propionibacterium und/oder der zumindest einen Art der Gattung Lactobacillus in dem ersten Kultivierungsprodukt, um ein zweites Kultivierungsprodukt zu erhalten, wobei das zweite Kultivierungsprodukt das vitamin- und proteinreiche Produkt ist und wobei das zweite Kultivierungsprodukt Biomasse der zumindest einen Art aus der Abteilung der Basidiomyceten und Biomasse der zumindest einen Vitamin B12-produzierenden Art der Gattung Propionibacterium und/oder Lactobacillus umfasst.

2. Verfahren nach Anspruch 1, wobei die zumindest eine Art aus der Abteilung der Basidiomyceten ausgewählt ist aus einer Gruppe bestehend aus *Agrocybe aegerita, Pleurotus roseus, Lentinula edodes, Laetiporus sulphureus, Pleurotus sapidus, Stropharia rugosoannulata* und/oder *Wolfiporia cocos.*

3. Verfahren nach einem der vorhergehenden Ansprüche, wobei die zumindest eine Vitamin B12-produzierende Art aus der Gattung Propionibacterium ausgewählt ist aus *Propionibacterium freudenreichii sups. freudenreichii* und/oder *Propionibacterium freudenreichii sups. Shermanii* und/oder wobei die zumindest eine Vitamin B12-produzierende Art aus der Gattung Lactobacillus *Lactobacillus reuteri* ist.

4. Verfahren nach einem der vorhergehenden Ansprüche, wobei das erste Kultivierungsprodukt eine Gesamtbiomasse im Bereich von 5 bis 45 g/L, vorzugsweise 10 bis 40 g/L, insbesondere vorzugsweise 15 bis 35 g/L, gemessen an der Trockenmasse, aufweist und/oder wobei das zweite Kultivierungsprodukt eine Gesamtbiomasse im Bereich von 10 bis 50 g /L, bevorzugt 15 bis 45 g /L, weiter bevorzugt 20 bis 40 g/L, gemessen an der Trockenmasse, aufweist.

5. Verfahren nach einem der vorhergehenden Ansprüche, wobei der zumindest eine kohlenhydrathaltige Agrarnebenstrom oder Lebensmittelnebenstrom ausgewählt ist aus der Gruppe bestehend aus Apfeltrester, Aroniatrester, Spinattrester, Granatapfeltrester, Rübenmelasse, Isomaltulosemelasse, Sonnenblumenkerntrester, Zwiebeltrester, Biertreber, Traubentrester, Heu und /oder Molke.

6. Verfahren nach einem der vorhergehenden Ansprüche, wobei das in Schritt a) eingesetzte Nährmedium aufweist: 5 bis 25 g/L Kohlenhydrate, vorzugsweise 10 bis 20 g/L Kohlenhydrate.

7. Verfahren nach einem der vorhergehenden Ansprüche, wobei das in Schritt a) eingesetzte Nährmedium weiterhin enthält: zumindest eine Stickstoffquelle, wobei die zumindest eine Stickstoffquelle vorzugsweise aus L-Asparagin, Ammoniumnitrat und/oder Hefeextrakt ausgewählt ist; zumindest eine Magnesiumquelle; zumindest eine Kaliumquelle und/oder eine Phosphatquelle; Spurenelemente, wobei die Spurenelemente Verbindungen von Eisen(II), Zink(II), Kupfer(II) und Mangan(II) umfassen.

8. Verfahren nach einem der vorhergehenden Ansprüche, wobei in Schritt b) die zumindest eine Vitamin B12-produzierende Art der Gattung Propionibacterium und/oder der Gattung Lactobacillus so zugegeben wird, dass eine Gesamtkeimzahl aller zugegebenen Bakterienarten in einem Bereich von $10^4$ bis $10^{10}$ CFU/ml, vorzugsweise $10^5$ bis $10^9$ CFU/ml, in dem ersten Kultivierungsprodukt vorliegt und/oder wobei das Kultivieren der zumindest einen Art der Gattung Propionibacterium und/oder der zumindest einen Art der Gattung Lactobacillus bis zum Erreichen einer Vitamin B12-Konzentration im Bereich von 1 bis 20 ng/ml Kultur, vorzugsweise 2 bis 15 ng/ml Kultur, insbesondere vorzugsweise 3 bis 10 ng/ml durchgeführt wird.

9. Verfahren nach einem der vorhergehenden Ansprüche 1-8, wobei das Verfahren weiterhin die Schritte umfasst:

d) Ernten der zumindest einen Art aus der Abteilung der Basidiomyceten und der zumindest einen Vitamin B12-produzierenden Art der Gattung Propionibacterium und/oder Lactobacillus aus dem zweiten Kultivierungsprodukt;
e) Trocknen der geernteten zumindest einen Art aus der Abteilung der Basidiomyceten und der zumindest einen Vitamin B12-produzierenden Art der Gattung Propionibacterium und/oder Lactobacillus zum Erhalten des vitamin- und proteinreichen Produkts.

10. Verfahren nach einem der vorhergehenden Ansprüche, wobei in Schritt b) weiterhin zumindest eine Glutaminaseaktive Bakterienart ausgewählt aus der Gattung Lactobacillus zugegeben wird, wobei die Glutaminase-aktive Bakterienart vorzugsweise *Lactobacillus rhamnosus* und/oder *Lactobacillus reuteri,* insbesondere vorzugsweise *Lactobacillus reuteri,* ist.

11. Verfahren nach einem der vorhergehenden Ansprüche, wobei das Verfahren ohne Ernten der zumindest einen Art aus der Abteilung der Basidiomyceten aus dem ersten Kultivierungsprodukt durchgeführt wird.

12. Vitamin- und proteinreiches Produkt herstellbar nach dem Verfahren einer der vorhergehenden Ansprüche.

13. Nahrungsmittel, vorzugweise Fleischersatzprodukt oder Tierfuttermittel, enthaltend das vitamin- und proteinreiche Produkt nach Anspruch 12.

14. Verwendung des vitamin- und proteinreichen Produkts nach Anspruch 13 zur Herstellung von Nahrungsmitteln, vorzugweise zur Herstellung von Fleischersatzprodukten und/oder Tierfuttermitteln.

**Claims**

1. A method for producing a vitamin- and protein-rich product, comprising the steps of:

   a) culturing at least one species from the Basidiomycetes division submerged in a nutrient medium containing at least one carbohydrate-containing agricultural sidestream or food sidestream to obtain a first culture product, wherein the first culture product comprises biomass of the at least one species from the Basidiomycetes division;
   b) adding at least one vitamin B12-producing species of the genus *Propionibacterium* and/or of the genus *Lactobacillus* to the first culture product;
   and
   c) culturing the at least one species of the genus *Propionibacterium* and/or the at least one species of the genus *Lactobacillus* in the first culture product to obtain a second culture product, wherein the second culture product is the vitamin- and protein-rich product and wherein the second culture product comprises biomass of the at least one species from the Basidiomycetes division and biomass of the at least one vitamin B12-producing species of the genus *Propionibacterium* and/or *Lactobacillus.*

2. The method according to claim 1, wherein the at least one species from the Basidiomycetes division is selected from a group consisting of *Agrocybe aegerita, Pleurotus roseus, Lentinula edodes, Laetiporus sulphureus, Pleurotus sapidus, Stropharia rugosoannulata* and/or *Wolfiporia cocos.*

3. The method according to any one of the preceding claims, wherein the at least one vitamin B12-producing species from the genus Propionibacterium is selected from *Propionibacterium freudenreichii sups. freudenreichii* and/or *Propionibacterium freudenreichii sups. Shermanii* and/or wherein the at least one vitamin B12-producing species from the genus *Lactobacillus* is *Lactobacillus reuteri.*

4. The method according to any one of the preceding claims, wherein the first cultivation product has a total biomass in the range of 5 to 45 g/L, preferably 10 to 40 g/L, particularly preferably 15 to 35 g/L, measured on the dry mass, and/or wherein the second cultivation product has a total biomass in the range of 10 to 50 g/L, preferably 15 to 45 g/L, more preferably 20 to 40 g/L, measured on the dry mass.

5. The method according to any one of the preceding claims, wherein the at least one carbohydrate-containing agricultural sidestream or food sidestream is selected from the group consisting of apple pomace, aronia pomace, spinach pomace, pomegranate pomace, beet molasses, isomaltulose molasses, sunflower seed pomace, onion pomace, beer pomace, grape pomace, hay and/or whey.

6. The method according to any one of the preceding claims, wherein the nutrient medium used in step a) has: 5 to 25 g/L carbohydrates, preferably 10 to 20 g/L carbohydrates.

7. The method according to any one of the preceding claims, wherein the nutrient medium used in step a) further contains: at least one nitrogen source, wherein the at least one nitrogen source is preferably selected from L-asparagine, ammonium nitrate and/or yeast extract; at least one magnesium source; at least one potassium source and/or a phosphate source; trace elements, wherein the trace elements comprise compounds of iron(II), zinc(II), copper(II) and manganese(II).

8. The method according to any one of the preceding claims, wherein in step b) the at least one vitamin B12-producing species from the genus *Propionibacterium* and/or the genus *Lactobacillus* is added such that a total germ count of all added bacterial species is in a range of $10^4$ to $10^{10}$ CFU/ml, preferably $10^5$ to $10^9$ CFU/ml, in the first culture product and/or wherein the culturing of the at least one species from the genus *Propionibacterium* and/or the at least one species from the genus *Lactobacillus* is carried out until reaching a vitamin B12 concentration in the range of 1 to 20 ng/ml culture, preferably 2 to 15 ng/ml culture, particularly preferably 3 to 10 ng/ml.

9. The method according to any one of the preceding claims 1-8, wherein the method further comprises the steps: d) harvesting the at least one species from the Basidiomycetes division and the at least one vitamin B12-producing species from the genus *Propionibacterium* and/or *Lactobacillus* from the second culture product; e) drying the harvested at least one species from the Basidiomycetes division and the at least one vitamin B12-producing species from the genus *Propionibacterium* and/or *Lactobacillus* to obtain the vitamin- and protein-rich product.

10. The method according to any one of the preceding claims, wherein in step b) at least one glutaminase-active bacterial species selected from the genus *Lactobacillus* is further added, wherein the glutaminase-active bacterial species is preferably *Lactobacillus rhamnosus* and/or *Lactobacillus reuteri,* particularly preferably *Lactobacillus reuteri.*

11. The method according to any one of the preceding claims, wherein the method is carried out without harvesting the at least one species from the Basidiomycetes division from the first culture product.

12. A vitamin- and protein-rich product producible according to the method of any one of the preceding claims.

13. A food, preferably meat substitute product or animal feed, comprising the vitamin- and protein-rich product according to claim 12.

14. Use of the vitamin- and protein-rich product according to claim 13 for producing food, preferably for producing meat substitute products and/or animal feed.


**Revendications**

1. Procédé de fabrication d'un produit riche en vitamines et en protéines, comprenant les étapes suivantes :

   a) culture d'au moins une espèce de la division des basidiomycètes submergés dans un milieu nutritif contenant au moins un sous-produit agricole ou un sous-produit alimentaire contenant des glucides, pour obtenir un premier produit de culture, le premier produit de culture comprenant une biomasse de l'au moins une espèce de la division des basidiomycètes ;
   b) addition d'au moins une espèce productrice de vitamine B12 du genre *Propionibacterium* et/ou du genre *Lactobacillus* au premier produit de culture ; et
   c) culture de l'au moins une espèce du genre *Propionibacterium* et/ou de l'au moins une espèce du genre *Lactobacillus* dans le premier milieu de culture, pour obtenir un deuxième produit de culture, le deuxième produit de culture étant le produit riche en vitamines et en protéines, le deuxième produit de culture comprenant une biomasse de l'au moins une espèce de la division des basidiomycètes et une biomasse de l'au moins une espèce productrice de vitamine B12 du genre *Propionibacterium* et/ou *Lactobacillus.*

2. Procédé selon la revendication 1, dans lequel l'au moins une espèce de la division des basidiomycètes est choisie dans un groupe constitué par *Agrocybe aegerita, Pleurotus roseus, Lentinula edodes, Laetiporus sulphureus, Pleurotus sapidus, Stropharia rugosoannulata* et/ou *Wolfiporia cocos.*

3. Procédé selon l'une des revendications précédentes, dans lequel l'au moins une espèce productrice de vitamine B12 du genre *Propionibacterium* est choisie parmi *Propionibacterium freudenreichii sups. Freudenreichii* et/ou *Propionibacterium freudenreichii sups. Shermanii* et/ou l'au moins une espèce productrice de vitamine B12 du genre *Lactobacillus* est *Lactobacillus reuteri.*

4. Procédé selon l'une des revendications précédentes, dans lequel le premier produit de culture présente une biomasse totale dans la plage de 5 à 45 g/l, de préférence de 10 à 40 g/l, en particulier de préférence de 15 à 35 g/l, mesurée sur la masse sèche, et/ou dans lequel le deuxième produit de culture présente une biomasse totale dans la plage de 10 à 50 g/l, de préférence de 15 à 45 g/l, plus préférentiellement de 20 à 40 g/l, mesurée sur la masse sèche.

5. Procédé selon l'une des revendications précédentes, dans lequel l'au moins un sous-produit agricole ou sous-produit alimentaire contenant des glucides est choisi dans le groupe constitué par le marc de pomme, le marc d'aronia, le marc d'épinard, le marc de grenade, la mélasse de betterave, la mélasse d'isomaltulose, le marc de graines de tournesol, le marc d'oignon, les drêches de brasserie, le marc de raisin, le foin et/ou le petit lait.

**6.** Procédé selon l'une des revendications précédentes, dans lequel le milieu nutritif utilisé à l'étape a) comprend 5 à 25 g/l de glucides, de préférence 10 à 20 g/l de glucides.

**7.** Procédé selon l'une des revendications précédentes, dans lequel le milieu nutritif utilisé à l'étape a) contient en outre :

au moins une source d'azote, l'au moins une source d'azote étant choisie de préférence parmi la L-asparagine, le nitrate d'ammonium et/ou l'extrait de levure ;
au moins une source de magnésium ;
au moins une source de potassium et/ou une source de phosphate ;
des oligoéléments, les oligoéléments comprenant des composés du fer (II), du zinc(II), du cuivre(II) et du manganèse(II).

**8.** Procédé selon l'une des revendications précédentes, dans lequel à l'étape b) l'au moins une espèce productrice de vitamine B12 du genre *Propionibacterium* et/ou du genre *Lactobacillus* est ajoutée de façon à avoir dans le premier produit de culture un nombre total de germes de la totalité des espèces de bactéries ajoutées compris dans une plage de $10^4$ à $10^{10}$ UFC/ml, de préférence de $10^5$ à $10^9$ UFC/ml, et/ou dans lequel la culture de l'au moins une espèce du genre *Propionibacterium* et/ou de l'au moins une espèce du genre *Lactobacillus* est mise en œuvre jusqu'à obtention d'une concentration de vitamine B12 comprise dans la plage de 1 à 20 ng/ml de culture, de préférence de 2 à 15 ng/ml de culture, en particulier de préférence de 3 à 10 ng/ml.

**9.** Procédé selon l'une des revendications précédentes 1 à 8, dans lequel le procédé comprend en outre les étapes suivantes :

d) récolte de l'au moins une espèce de la division des basidiomycètes et de l'au moins une espèce productrice de vitamine B12 du genre *Propionibacterium* et/ou *Lactobacillus,* à partir du deuxième produit de culture ;
e) séchage de l'au moins une espèce récoltée de la division des basidiomycètes et de l'au moins une espèce productrice de vitamine B12 du genre *Propionibacterium* et/ou *Lactobacillus* jusqu'à obtention du produit riche en vitamines et en protéines.

**10.** Procédé selon l'une des revendications précédentes, dans lequel à l'étape b) on ajoute en outre au moins une espèce de bactérie à activité de glutaminase, choisie parmi le genre *Lactobacillus,* l'espèce de bactérie à activité de glutaminase étant de préférence *Lactobacillus rhamnosus* et/ou *Lactobacillus reuteri,* en particulier de préférence *Lactobacillus reuteri.*

**11.** Procédé selon l'une des revendications précédentes, dans lequel le procédé est mis en œuvre sans récolte de l'au moins une espèce de la division des basidiomycètes à partir du premier produit de culture.

**12.** Produit riche en vitamines et en protéines pouvant être fabriqué par le procédé selon l'une des revendications précédentes.

**13.** Produit alimentaire, de préférence produit substitut de viande ou aliment pour le bétail, contenant le produit riche en vitamines et en protéines selon la revendication 12.

**14.** Utilisation du produit riche en vitamines et en protéines selon la revendication 13 pour la fabrication de produits alimentaires, de préférence pour la fabrication de produits substituts de viande et/ou d'aliments pour le bétail.

Fig. 1

b)

Fig. 2

a)

b)

Fig. 3

a)

b)

Fig. 4

Fig. 5

Fig. 6

E.coli ······ E.coli ——— Salmonella enteridis – – Salmonella enteridis ——— Listeria – –Listeria ▬▬▬ Pseudomonas ---- Pseudomonas monocytogenes monocytogenes aeruginosa aeruginosa

* p< 0,05

Fig. 7

$y = -1,28x + 4,0519$
$R^2 = 0,9508$

Fig. 8

Fig. 9

Fig. 10

Fig. 11

⊠Protein ☐KH ⊡Asche

Fig. 12

△ PSA Apfel Lyophilisat  o PSA Apfel flüssig

Belichtungsdauer [min]

Fig. 13

Fig. 14

Fig. 15

Fig. 16

Fig. 17

Fig. 18

Fig. 19

Fig. 20

Fig. 21

Fig. 22

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1094719 B1
- US 20090148558 A
- DE 202010016402 U1
- EP 2580316 A2